# EUROPEAN PATENT SPECIFICATION

(11) **EP 2 606 150 B1**
(45) Date of publication and mention of the grant of the patent: **16.03.2016**
(21) Application number: 11755471.7
(22) Date of filing: 19.08.2011
(51) Int. Cl.: C12Q 1/68

(54) **QUANTITATIVE REAL-TIME PCR ASSAY USING FRET DUAL-LABELED PRIMERS**
QUANTITATIVER ECHTZEIT-PCR-TEST MIT DOPPELMARKIERTEN FRET-PRIMERN
DOSAGE D'AMPLIFICATION EN CHAÎNE PAR POLYMÉRASE EN TEMPS RÉEL UTILISANT DES AMORCES À DOUBLE MARQUAGE DE TRANSFERT D'ÉNERGIE PAR RÉSONANCE DE FLUORESCENCE

(30) Priority: 20.08.2010 US 375318 P
(43) Date of publication of application: 26.06.2013
(73) Proprietor: Life Technologies Corporation, Carlsbad, CA 92008 (US)
(72) Inventor: GE, Wayne, Austin Texas 78739 (US); NG, Yue Ling, Singapore 350106 (SG)
(74) Representative: Weber, Birgit
(86) International application number: PCT/US2011/048506
(87) International publication number: WO 2012/024642

(56) References cited:
- WO-A1-2008/063194
- WO-A1-2010/018245
- WO-A1-2011/078441
- SOLINAS A ET AL: "Duplex Scorpion primers in SNP analysis and FRET applications.", NUCLEIC ACIDS RESEARCH 15 OCT 2001 LNKD- PUBMED:11600715, vol. 29, no. 20, 15 October 2001 (2001-10-15), page E96, XP002318909, ISSN: 1362-4962
- LIU ET AL: "Q-priming PCR: A quantitative real-time PCR system using a self-quenched BODIPY FL-labeled primer", ANALYTICAL BIOCHEMISTRY, ACADEMIC PRESS INC, NEW YORK, vol. 360, no. 1, 14 December 2006 (2006-12-14), pages 154-156, XP005725318, ISSN: 0003-2697, DOI: 10.1016/J.AB.2006.10.011
- JORDENS J Z ET AL: "Amplification with molecular beacon primers and reverse line blotting for the detection and typing of human papillomaviruses", JOURNAL OF VIROLOGICAL METHODS, ELSEVIER BV, NL, vol. 89, no. 1-2, 1 September 2000 (2000-09-01), pages 29-37, XP002250695, ISSN: 0166-0934, DOI: 10.1016/S0166-0934(00)00195-6

## Description

### CROSS-REFERENCE TO RELATED APPLICATIONS

This application claims the benefit of priority under 35 U.S.C. §119(e) to Provisional U.S. Application No. 61/375,318, filed August 20, 2010.

### FIELD

This specification generally relates to non-radioactive methods of real-time PCR using fluorescence resonance energy transfer (FRET) dual-labeled primers.

### BACKGROUND

Fluorescence resonance energy transfer (FRET) is a form of molecular energy transfer (MET), a process by which energy is passed non-radioactively between a donor molecule and an acceptor molecule. FRET arises from the properties of certain chemical compounds; when excited by exposure to particular wavelengths of light, they emit light (i.e., they fluoresce) at a different wavelength. Such compounds are termed fluorophores. In FRET, energy is passed non-radioactively over a long distance (e.g., 10-100 Angstroms) between a donor molecule, which is a fluorophore, and an acceptor molecule, which is a quencher. The donor absorbs a photon and transfers this energy non-radioactively to the acceptor (Forster, 1949, Z. Naturforsch. A4: 321-327; Clegg, 1992, Methods Enzymol. 211: 353-388).

When two fluorophores whose excitation and emission spectra overlap are in close proximity, excitation of one fluorophore will cause it to emit light at wavelengths that are absorbed by and that stimulate the second fluorophore, causing it in turn to fluoresce. In other words, the excited-state energy of the first (donor) fluorophore is transferred by a resonance induced dipole-dipole interaction to the neighboring second (acceptor) fluorophore. As a result, the lifetime of the donor molecule is decreased and its fluorescence is quenched, while the fluorescence intensity of the acceptor molecule is enhanced and depolarized. When the excited-state energy of the donor is transferred to a non-fluorophore acceptor, the fluorescence of the donor is quenched without subsequent emission of fluorescence by the acceptor. In this case, the acceptor functions as a quencher.

Pairs of molecules that can engage in FRET are termed FRET pairs. In order for energy transfer to occur, the donor and acceptor molecules must typically be in close proximity (e.g., up to 70 to 100 Angstroms) (Clegg, 1992, Methods Enzymol. 211: 353-388; Selvin, 1995, Methods Enzymol. 246: 300-334). The efficiency of energy transfer falls off rapidly with the distance between the donor and acceptor molecules. Effectively, this means that FRET can most efficiently occur up to distances of about 70 Angstroms.

Commonly used methods for detecting nucleic acid amplification products require that the amplified product be separated from unreacted primers. This is commonly achieved either through the use of gel electrophoresis, which separates the amplification product from the primers on the basis of a size differential, or through the immobilization of the product, allowing washing away of free primer. Other methods treat the amplification product with a 3'→5' exonuclease to digest free primers or by heating the amplification product to a temperature such that the oligonucleotide-primer duplex dissociates and, as a result, will not generate any signal (U.S. Patent No. 5,866,336). Other methods for monitoring the amplification process without prior separation of primer have been described. Some examples include TaqMan^{®} probes, molecular beacons, SYBR Green^{®} indicator dye, LUX primers, and others. All current assays that utilize FRET rely on the physical cleavage of the fluorophore from the quencher for detection of the amplification and some require an additional probe to identify the target sequence.

One method for detection of amplification product without prior separation of primer and product is the 5' nuclease PCR assay (also referred to as the TaqMan^{®} assay) (Holland et al., 1991, Proc. Natl. Acad. Sci. USA 88: 7276-7280; Lee et al., 1993, Nucleic Acids Res. 21: 3761-3766). This assay detects the accumulation of a specific PCR product by hybridization and cleavage of a doubly-labeled fluorogenic probe (the "TaqMan^{®}" probe) during the amplification reaction. The fluorogenic probe consists of an oligonucleotide labeled with both a fluorescent reporter dye and a quencher dye. During PCR, this probe is cleaved by the 5'-exonuclease activity of DNA polymerase if, and only if, it hybridizes to the segment being amplified. Cleavage of the probe generates an increase in the fluorescence intensity of the reporter dye.

Another method of detecting amplification products that relies on the use of energy transfer is the "molecular beacon probe" method described by Tyagi and Kramer (1996, Nature Biotech. 14:303-309) which is also the subject of U.S. Pat. Nos. 5,119,801 and 5,312,728 to Lizardi et al. This method employs oligonucleotide hybridization probes that can form hairpin structures. On one end of the hybridization probe (either the 5' or 3' end) there is a donor fluorophore, and on the other end, an acceptor moiety. In the case of the Tyagi and Kramer method, this acceptor moiety is a quencher, that is, the acceptor absorbs energy released by the donor, but then does not itself fluoresce. Thus when the beacon is in the open conformation, the fluorescence of the donor fluorophore is detectable, whereas when the beacon is in the hairpin (closed) conformation, the fluorescence of the donor fluorophore is quenched. When employed in PCR, the molecular beacon probe, which hybridizes to one of the strands of the PCR product, is in the "open conformation," and fluorescence is detected, while those that remain unhybridized will not fluoresce (Tyagi and Kramer, 1996, Nature Biotechnol. 14: 303-306). As a result, the amount of fluorescence will increase as the amount of PCR product increases, and thus may be used as a measure of the progress of the PCR.

WO201001824 discloses in Figs. 1 and 5 oligonuckeotides which are labeled with fluorophore and quencher for realtime detection. Once the 3'label is cleaved off by a proofreading activity the oligonucleotide can be extended as a primer. However, the method relies on the G or C residues to either quench or enhance the fluorescence signal.

Solinas et al. disclose in Nucl. Acids Res. 2001, Vol. 29, No. 20 e96 scorpion oligonucleotides which are PCR primers , which contain a stemloop portion containing the fluorophore and the quencher, and the sequence between the fluorophore and the probe is not complementary to any part of the target nucleic acid.

Liu et al. disclose in Analytical Biochemistry 2006, Vol. 360, 154-156 a primer having a fluorophor quenched by guanine residues a the 3'end of the hairpin. It relies upon cleavage of the oligonucleotide by 5'-3-exonuclease activity to physically separate the fluorophore and quencher and thereby generate a signal.

Jordans et al. describe in J. Virol. Meth. 2000, Vol. 89, 29-37, a real-time detection method using a molecular beacon. The beacon does not contain any of the complementary sequence between the nucleotides that the fluorophore and the quencher are attached to,

WO2008063194 describes dual function primers which fluorescence-quenched oligonucleotide that can prime DNA extension reactions, and function as a template for DNA extension reactions. Signal detection is dependent upon DNA synthesis and can occur with or without probe cleavage

Because most of these and other known methods using fluorescent primers require both primers and a probe, they are not always useful in the amplification of very small amplicons. Therefore, in view of the state of the art, a need exists for broadly applicable assays for PCR using a non-radioactive method that can also be used for very small targets. The improvements needed involve primer design flexibility, better target detection sensitivity, faster annealing and extension, and expanded PCR applications for mutation/SNP/subtype PCR and multiplex PCR.

The subject matter discussed in the background section should not be assumed to be prior art merely as a result of its mention in the background section. Similarly, a problem mentioned in the background section or associated with the subject matter of the background section should not be assumed to have been previously recognized in the prior art. The subject matter in the background section merely represents different approaches, which in and of themselves may also be inventions.

### SUMMARY

Any of the above embodiments may be used alone or together with one another in any combination. Inventions encompassed within this specification may also include embodiments that are only partially mentioned or alluded to or are not mentioned or alluded to at all in this brief summary or in the abstract.

Some aspects include methods for quantifying or detecting one or more target nucleic acid molecules in a sample during nucleic acid synthesis comprising:
mixing one or more target nucleic acid molecules with one or more fluorescently labeled oligonucleotide primers complementary to a portion of said one or more target nucleic acid molecules, wherein the one or more oligonucleotides are labeled with a fluorophore and a quencher wherein one or both of the fluorophore and quencher are internally located in the primer and wherein the Quencher is located near or at the 5'-end and the oligonucleotide undergoes a detectable change in fluorescence upon extension of the one or more target nucleic acid molecules;
incubating the mixture with a polymerase under conditions sufficient to synthesize one or more nucleic acid molecules complementary to all or a portion of the one or more target nucleic acid molecules, the one or more synthesized nucleic acid molecules comprising the one or more oligonucleotides; and
detecting the presence or absence and/or quantifying the amount of the one or more synthesized nucleic acid molecules by measuring the fluorophore, wherein the extension is by at least 3 nucleotides (i.e., at least three nucleotides are added during the synthesizing steps). In some embodiments, the steps may be performed simultaneously or separately in any order.

In some embodiments, the quencher and fluorophore are separated at a distance such that when the duplex is not polymerized the fluorophore is quenched by the quencher and when the duplex is polymerized the fluorophore is not quenched by the quencher. In some embodiments, the fluorophore and quencher are between about 3 nucleotides and about 20 nucleotides apart on the same oligonucleotide. In some embodiments, the distance is between about 6 nucleotides and about 19 nucleotides. In some embodiments, the fluorophore is chosen from fluorescein, 5-carboxyfluorescein (FAM), 2'7'-dimethoxy-4'5'-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), and 5-(2'-aminoethyl)aminonaphthalene-1-sulfonic acid (EDANS). In some embodiments, the quencher is chosen from: a Black Hole Quencher^{®}, an IOWA Black^{®} quencher, an Eclipse^{®} Dark quencher and a DABCYL quencher and a derivative thereof. In some embodiments, the fluorophore is internal and the quencher is on the 5' end of the oligonucleotide. In some embodiments, the quencher is internal and the fluorophore is on the 5' end of the oligonucleotide. In some embodiments, target nucleic acid is from about 15 nucleotides to about 100 nucleotides in length. In some embodiments, the detection is performed using a spectrophotometric real-time PCR instrument. In some embodiments, the target nucleic acid is chosen from genomic DNA, RNA, cDNA, mRNA, and chemically synthesized DNA. In some embodiments, the target nucleic acid is a sequence of an infectious disease agent. In some embodiments, the target nucleic acid is a wild-type human genomic sequence, or a mutation implicated in a human disease or disorder. In some embodiments, the method also includes denaturing the product and incubating under conditions sufficient to synthesize one or more nucleic acid molecules complementary to all or a portion of the one or more target nucleic acid molecules, the one or more synthesized nucleic acid molecules comprising the one or more oligonucleotides. In some embodiments, the method includes repeating the denaturing and incubating one or more times.

Other embodiments provide methods of amplifying double-stranded nucleic acid molecules comprising:
providing at least a first and a second primer, wherein the first primer is complementary to a sequence within or at or near the 3'-terminus of the first strand of the nucleic acid molecule and the second primer is complementary to a sequence within or at or near the 3'-terminus of the second strand of the nucleic acid molecule;
hybridizing the first primer to the first strand and the second primer to the second strand in the presence of one or more polymerases, under conditions such that the primers are extended to result in the synthesis of a third nucleic acid molecule complementary to all or a portion of the first strand and a fourth nucleic acid molecule complementary to all or a portion of the second strand;
denaturing the first and third strands, and the second and fourth strands; and
repeating the above steps one or more times, wherein one of the first and second primers is dual-labeled with a fluorophore and a quencher; ; wherein one or both of the fluorophore and quencher are internally located in the primer and wherein the Quencher is located near or at the 5'-end and
wherein the dual-labeled primer undergoes a detectable change in fluorescence upon extension of the one or more labeled primers to the nucleic acid molecule, wherein the extension is by at least 3 nucleotides. In some embodiments, the steps may be performed simultaneously or separately in any order. In some embodiments, the quencher and fluorophore are separated at a distance such that when the duplex is not polymerized the fluorophore is quenched by the quencher and when the duplex is polymerized the fluorophore is not quenched by the quencher. In some embodiments, the fluorophore and quencher are between about x and y nucleotides apart on the same oligonucleotide. In some embodiments, the distance is between about 4 nucleotides and about 20 nucleotides. In some embodiments, the fluorophore is chosen from fluorescein, 5-carboxyfluorescein (FAM), 2'7'-dimethoxy-4'5'-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), and 5-(2' aminoethyl) aminonaphthalene-1-sulfonic acid (EDANS). In some embodiments, the quencher is chosen from: a Black Hole Quencher^{®}, an Iowa Black^{®} quencher, an Eclipse^{®} Dark quencher, a DABCYL quencher and derivatives thereof. In some embodiments, the fluorophore is internal and the quencher is on the 5' end of the oligonucleotide. In some embodiments, the target nucleic acid is from about 15 to about 100 nucleotides in length. In some embodiments, the detection is performed using a real-time PCR instrument. In some embodiments, the target nucleic acid is chosen from genomic DNA, RNA, cDNA, mRNA, and chemically synthesized DNA. In some embodiments, the target nucleic acid is a sequence of an infectious disease agent. In some embodiments, the target nucleic acid is a wild-type human genomic sequence, or a mutation implicated in a human disease or disorder.

Further embodiments provide for compositions comprising a dual-labeled FRET primer comprising an oligonucleotide, wherein the oligonucleotide is labeled with both a fluorophore and a quencher and the oligonucleotide undergoes a detectable change in fluorescence upon extension by at least three nucleotidesThe quencher and fluorophore are separated at a distance such that when the duplex is not polymerized the fluorophore is quenched by the quencher and when the duplex is polymerized the fluorophore is not quenched by the quencherThe distance is between about 3 nucleotides and about 20 nucleotides. In some embodiments, the fluorophore is chosen from fluorescein, 5-carboxyfluorescein (FAM), 2'7'-dimethoxy-4'5'-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), and 5-(2' aminoethyl) aminonaphthalene-1-sulfonic acid (EDANS). In some embodiments, the quencher is chosen from: a Black Hole Quencher^{®}, an Iowa Black^{®} quencher, an Eclipse^{®} Dark quencher, a DABCYL quencher and derivatives thereof. In some embodiments, the fluorophore is internal and the quencher is on the 5' end of the oligonucleotide.

Further embodiments provide for kits for the quantification and/or detection of one or more target nucleic acid molecules in a sample during nucleic acid synthesis, including a polymerase, and a dual-labeled oligonucleotide comprising a fluorophore and a quencher, wherein the quencher and fluorophore are separated at a distance such that when the duplex is not polymerized the fluorophore is quenched by the quencher and when polymerized the fluorophore is not quenched by the quencher. The distance is between about 3 nucleotides and about 20 nucleotides.

### BRIEF DESCRIPTION OF THE FIGURES

Although the following figures depict various examples of the invention, the invention is not limited to the examples depicted in the figures.
Figure 1 depicts real time quantification of serially diluted equine herpes virus (EHV1) DNA by FRET primers (only reverse primer is FRET labeled) in comparison to a Taqman^{®} assay. A: EHV1 target DNA (SEQ ID NO:1) and primer probe sequences. B: FRET primer displayed similar quantification over a range of 10,000 to 50 copies of EHV1 template DNA when compared to the Taqman^{®} assay.
Figure 2 is a graph showing an assay identifying whether the FRET primer assay involves the use of the 5'→3' exonuclease activity of Taq.
Figure 3 is a graph showing the dissociation curve analysis of amplified product with FRET primer from Example 2.
Figures 4A-C show the dependence of the FRET primer assay for real-time detection of target DNA on extension. FIG 4A shows the FRET primers GA445 forward (SEQ ID NO:2) and GA438 reverse (SEQ ID NO:3). FIG 4B shows a multicomponent plot of the assay for 0, 1, 2, 4 and 5 nt extensions. FIG 4C is a graph showing a melting curve of the amplified products primed with FRET primers.
Figure 5 shows that the FRET primer assay is different from other assays which rely on the secondary structures of primers or probe. Figure 5A shows the amplification plot and Figure 5B shows the melting curve analysis for the FRET primer assay as compared to the molecular beacon.
Figures 6A-C show that FRET primer assays provide better amplicon size differentiation by melting curve analysis than SYBR Green^{®} assays. FIG 6A shows the fluorescence peak for amplicon sizes 24-564 with FRET primers. FIG 6B shows the fluorescence peak for amplicon sizes 24-564 for SYBR Green^{®} primers. FIG 6C shows the Tm dC by dissociation for FRET primers and SYBR Green^{®} primers as a function of the amplicon size.
Figure 7 shows that the FRET primer assay can be performed using shorter annealing and extension time than the TaqMan^{®} assay.
Figure 8 shows that the FRET primer assay can be used successfully with different PCR reagent systems. Figure 8A shows the amplification plot and Figure 8B shows the melting curve analysis for the FRET primer assay using PCR reagent systems from Qiagen RT-PCR, Qiagen PCR, AB Uni PCR, and AgPath-ID™ PCR.

### DETAILED DESCRIPTION

Although various embodiments of the invention may have been motivated by various deficiencies with the prior art, which may be discussed or alluded to in one or more places in the specification, the embodiments of the methods, compositions and kits disclosed herein do not necessarily address any of these deficiencies. In other words, different embodiments disclosed herein may address different deficiencies that may be discussed in the specification. Some embodiments may only partially address some deficiencies or just one deficiency that may be discussed in the specification, and some embodiments may not address any of these deficiencies.

In general, the specification provides methods and compositions for the Polymerase Chain Reaction (PCR) using non-radioactive methods. The non-radioactive methods disclosed herein involve real-time PCR using FRET dual-labeled primers and do not require the use of a probe. The non-radioactive methods may also be used for end-point PCR in which the signal is measured only at the endpoint of the PCR cycling. The dual-labeled primer maintains the molecular tether between fluorophore and quencher. When PCR is carried out with the dual-labeled primer, the extension of the primer by a polymerase by at least 3 nucleotides releases fluorescence. Without being restricted to a specific mechanism, the fluorescence is released presumably by forcing the fluorophore-quencher pair apart by the rigidity of the double-stranded structure. The methods disclosed herein reveal a new mechanism to utilize FRET relying on the extension of dual labeled primers and the formation of a duplex structure. Further, the methods waive the requirement of a separate probe targeted to the middle of the amplicon and provide valuable flexibility in designing primers and assays. The methods are particularly useful for assays targeting highly mutated nucleic acids, such as RNA viral genes, and short fragments, such as miRNA, piRNA and siRNA. Since the assays do not require a probe, short fragments that do not have enough length for the design of a pair of primers and a probe may be detected and/or quantified with the methods provided herein. Thus, the methods are also useful for targets that may be partially degraded and or fragmented, such as forensic samples and fixed tissues.

The methods provided herein are also partially based on the surprising discovery that when PCR is carried out with one primer dual-labeled with a fluorophore and a quencher (one internal and the other 5' terminal), the extension of the primer by a DNA polymerase by at least 3 nucleotides releases fluorescence. The increase in fluorescence increases with the amount of extended primers in a direct relationship.

The methods provided herein are also partially based on the surprising discovery that the distance between the fluorophore and quencher corresponds to the amount of background fluorescence from unincorporated primers. It was unexpectedly discovered that a distance of between about 3 and 20 nucleotides resulted in efficient quenching of the fluorophore when the primer is unincorporated and an increase in fluorescence upon incorporation into the amplified product. By having the fluorophore and quencher separated by this distance and on the same oligonucleotide obviates the need to perform additional treatment of the amplified product to remove unicorporated primers and thereby remove background fluorescence.

The methods provided herein provide several advantages over existing methods, including reducing the time to result, simplifying the workflow, eliminating time consuming steps, eliminating the need for separating or removing the unincorporated primers and reducing costs. The methods provide improvements for quantitative real-time nucleic acid amplification by enabling primer design flexibility, better target detection sensitivity, faster annealing and extension, and expanded PCR applications for mutation/SNP (single nucleotide polymorphism)/subtype PCR and multiplex PCR.

### Definitions and General Methods:

The practice of the present invention will employ, unless otherwise indicated, conventional methods of chemistry, biochemistry, molecular biology, virology, immunology and pharmacology, within the skill of the art. Such techniques are explained fully in the literature.

In the description that follows, a number of terms used in chemistry, biochemistry, molecular biology, virology, immunology and pharmacology are extensively utilized. In order to provide a clearer and consistent understanding of the specification and claims, including the scope to be given such terms, the following definitions are provided.

As used in this specification and the appended claims, the singular forms "a," "an" and "the" include plural references unless the content clearly dictates otherwise.

The terms "nucleic acid," "polynucleotide," "oligonucleotide" or "oligo" mean polymers of nucleotide monomers or analogs thereof, including double- and single-stranded deoxyribonucleotides, ribonucleotides, alpha-anomeric forms thereof, and the like. Usually, the monomers are linked by phosphodiester linkages, where the term "phosphodiester linkage" refers to phosphodiester bonds or bonds including phosphate or analogs thereof, including associated counterions, e.g., H⁺, NH₄⁺, Na⁺.

As used herein "nucleotide" refers to a base-sugar-phosphate combination. Nucleotides are monomeric units of a nucleic acid sequence (DNA and RNA) and deoxyribonucleotides are "incorporated" into DNA by DNA polymerases. The term nucleotide includes deoxyribonucleoside triphosphates such as dATP, dCTP, dITP, dUTP, dGTP, dTTP, or derivatives thereof. Such derivatives include, for example, [aS]dATP, 7-deaza-dGTP and 7-deaza-dATP. The term nucleotide as used herein also refers to dideoxyribonucleoside triphosphates (ddNTPs) and their derivatives. Illustrated examples of dideoxyribonucleoside triphosphates (ddNTPs) include, but are not limited to, ddATP, ddCTP, ddGTP, ddITP, and ddTTP.

The term "nucleic acid or nucleotide analogs" refers to analogs of nucleic acids made from monomeric nucleotide analog units, and possessing some of the qualities and properties associated with nucleic acids. Nucleotide analogs may have modified (i) nucleobase moieties, e.g. C-5-propyne pyrimidine, pseudo-isocytidine and isoguanosine, (ii) sugar moieties, e.g. 2'-O-alkyl ribonucleotides, and/or (iii) internucleotide moieties, e.g. 3'-N-phosphoramidate. See Englisch, U. and Gauss, D. "Chemically modified oligonucleotides as probes and inhibitors", Angew. Chem. Int. Ed. Engl. 30:613-29 (1991). A class of analogs where the sugar and internucleotide moieties have been replaced with a 2-aminoethylglycine amide backbone polymer is peptide nucleic acids PNA. See P. Nielsen et al., Science 254:1497-1500 (1991).

As used herein, the terms "hybridization" and "hybridizing" refer to the pairing of two complementary single-stranded nucleic acid molecules (RNA and/or DNA) to give a double-stranded molecule. As used herein, two nucleic acid molecules may be hybridized, although the base pairing is not completely complementary. Accordingly, mismatched bases do not prevent hybridization of two nucleic acid molecules provided that appropriate conditions, well known in the art, are used.

The term "end-point" measurement refers to a method where data collection occurs only once the reaction has been stopped.

The term "real-time" and "real-time continuous" are interchangeable and refer to a method where data collection occurs through periodic monitoring during the course of the polymerization reaction. Thus, the methods combine amplification and detection into a single step.

As used herein, the term "quantitative PCR" refers to the use of PCR to quantify gene expression.

As used herein, the terms "Cₜ" and "cycle threshold" refer to the time at which fluorescence intensity is greater than background fluorescence. They are characterized by the point in time (or PCR cycle) where the target amplification is first detected. Consequently, the greater the quantity of target DNA in the starting material, the faster a significant increase in fluorescent signal will appear, yielding a lower Cₜ.

As used herein, the term "amplification" refers to any *in vitro* method for increasing the number of copies of a nucleotide sequence with the use of a polymerase. Nucleic acid amplification results in the incorporation of nucleotides into a nucleic acid (e.g., DNA) molecule or primer thereby forming a new nucleic acid molecule complementary to the nucleic acid template. The newly formed nucleic acid molecule and its template may be used as templates to synthesize additional nucleic acid molecules. As used herein, one amplification reaction may consist of many rounds of nucleic acid synthesis. Amplification reactions include, for example, polymerase chain reactions (PCR). One PCR reaction may consist of 5 to 100 "cycles" of denaturation and synthesis of a nucleic acid molecule.

The term "incorporating" as used herein means becoming a part of a DNA or RNA molecule or primer.

As used herein, the term "primer" refers to a synthetic or biologically produced single-stranded oligonucleotide that is extended by covalent bonding of nucleotide monomers during amplification or polymerization of a nucleic acid molecule. Nucleic acid amplification often is based on nucleic acid synthesis by a nucleic acid polymerase or reverse transcriptase. Many such polymerases or reverse transcriptases require the presence of a primer that may be extended to initiate such nucleic acid synthesis. As will be appreciated by those skilled in the art, the oligonucleotides of the invention may be used as one or more primers in various extension, synthesis or amplification reactions.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% complementary refers to the situation in which each nucleotide unit of one polynucleotide strand or region can hydrogen bond with each nucleotide unit of a second polynucleotide strand or region. "Less than perfect complementarity" refers to the situation in which some, but not all, nucleotide units of two strands or two regions can hydrogen bond with each other.

As used herein, the term "reverse complement" or "RC" refers to a sequence that will anneal/base pair or substantially anneal/base pair to a second oligonucleotide according to the rules defined by Watson-Crick base pairing and the antiparallel nature of the DNA-DNA, RNA-RNA, and RNA-DNA double helices. Thus, as an example, the reverse complement of the RNA sequence 5'-AAUUUGC would be 5'GCAAAUU. Alternative base pairing schemes including but not limited to G-U pairing can also be included in reverse complements.

As used herein, the term "probe" refers to synthetic or biologically produced nucleic acids (DNA or RNA) which, by design or selection, contain specific nucleotide sequences that allow them to hybridize, under defined stringencies, specifically (i.e., preferentially) to target nucleic acid sequences.

As used herein, the term "template" is interchangeable with "target molecule" and refers to a double-stranded or single-stranded nucleic acid molecule which is to be amplified, copied or extended, synthesized or sequenced. In the case of a double-stranded DNA molecule, denaturation of its strands to form a first and a second strand is performed to amplify, sequence or synthesize these molecules. A primer, complementary to a portion of a template is hybridized under appropriate conditions and the polymerase (DNA polymerase or reverse transcriptase) may then synthesize a nucleic acid molecule complementary to said template or a portion thereof. The newly synthesized molecule, according to the invention, may be equal or shorter in length than the original template. Mismatch incorporation during the synthesis or extension of the newly synthesized molecule may result in one or a number of mismatched base pairs. Thus, the synthesized molecule need not be exactly complementary to the template. The template may be an RNA molecule, a DNA molecule or an RNA/DNA hybrid molecule. A newly synthesized molecule may serve as a temp late for subsequent nucleic acid synthesis or amplification.

The term "target molecule", as used herein, refers to a nucleic acid molecule to which a particular primer or probe is capable of preferentially hybridizing.

The term "target sequence", as used herein, refers to a nucleic acid sequence within the target molecules to which a particular primer is capable of preferentially hybridizing.

As used herein, the term "thermostable" refers to a polymerase (RNA, DNA or RT) which is resistant to inactivation by heat. DNA polymerases synthesize the formation of a DNA molecule complementary to a single-stranded DNA template by extending a primer in the 5'-to-3' direction. This activity for mesophilic DNA polymerases may be inactivated by heat treatment. For example, T5 DNA polymerase activity is totally inactivated by exposing the enzyme to a temperature of 90°C for 30 seconds. As used herein, a thermostable DNA polymerase activity is more resistant to heat inactivation than a mesophilic DNA polymerase. However, a thermostable DNA polymerase does not mean to refer to an enzyme which is totally resistant to heat inactivation and thus heat treatment may reduce the DNA polymerase activity to some extent. A thermostable DNA polymerase typically will also have a higher optimum temperature than mesophilic DNA polymerases.

As used herein, the term "additional treatments" refers to procedures used to separate or remove the unincorporated, or free, primer from the amplification product. Such additional treatments include, but are not limited to, gel electrophoresis, immobilization of the amplification product and washing away the free primer, digestion of the unincorporated primer, such as by incubation with a 3'→5' exonuclease, heat treatment to dissociate the free primer, and denaturation of the primer.

As used herein, the terms "fluorophore," "fluorescent moiety," "fluorescent label" and "fluorescent molecule" are interchangeable and refer to a molecule, label or moiety that has to absorb energy from light, transfer this energy internally, and emit this energy as light of a characteristic wavelength.

As used herein, the terms "quencher," "quencher moiety," and "quencher molecule" are interchangeable and refer to a molecule, moiety, or label that is capable of quenching a fluorophore emission. This can occur as a result of the formation of a non-fluorescent complex between the fluorophore and the quencher.

### Methods

In general, the specification provides methods and compositions for polymerase chain reaction (PCR) using non-radioactive methods. The non-radioactive methods disclosed herein involve real-time PCR using FRET dual-labeled primers. The methods may also be used for quantitative PCR. The FRET dual-labeled primer maintains the molecular tether between fluorophore and quencher. When PCR is carried out with the dual-labeled primer, the extension of the primer by a polymerase by at least 3 nucleotides releases fluorescence. Without being restricted to a specific mechanism, the fluorescence is released presumably by forcing the fluorophore-quencher (fluor-quench) pair apart by the rigidity of the double-stranded structure. The methods provided herein reveal a new mechanism to utilize FRET relying on the extension of dual-labeled primers and the formation of a duplex structure. Further, the methods waive the requirement of a separate probe targeted to the middle of the amplicon and provide valuable flexibility in designing primers and assays. In addition, the methods obviate the need for additional treatment of the amplification product to remove or separate the amplification product from unincorporated, or free, primer. The methods are particularly useful for assays targeting highly mutated nucleic acids, such as RNA viral genes, and short fragments, such as siRNA and miRNA, fragmented or denatured samples (such as forensic samples). Since the assays do not require a probe, short fragments that do not have enough length for the design of a pair of primers and a probe can be detected and/or quantified with the methods. In some embodiments, the RT-PCR is carried out in real time and in a quantitative manner. Real time quantitative RT-PCR has been thoroughly described in the literature (see Gibson, et al., Genome Res. 1996. 6: 995-1001 for an early example of the technique).

Real-time PCR techniques produce a fluorescent read-out that can be continuously monitored. Real-time techniques are advantageous because they keep the reaction in a "single tube". This means there is no need for downstream analysis in order to obtain results, leading to more rapidly obtained results. Furthermore, keeping the reaction in a "single tube" environment reduces the risk of cross contamination and allows a quantitative output from the methods disclosed herein. This may be particularly important in clinical settings. The theory and methods of real-time and quantitative PCR are known to those of skill in the art, are also reviewed, for example, in "Real-time PCR for mRNA quantitation" BioTechniques (2005) 39, No.1, pages 1-11.

It should be noted that PCR is one amplification method that can be used with the FRET primer assay disclosed herein. Variations on the basic PCR technique such as nested PCR or other equivalent methods may also be included within the scope of this disclosure. Examples include isothermal amplification techniques such as NASBA, 3SR, TMA and triamplification, all of which are well known in the art and commercially available. Other suitable amplification methods include the ligase chain reaction (LCR) (Barringer et al, Gene 89:117-122 (1990)), selective amplification of target polynucleotide sequences (U.S. Pat. No. 6,410,276), consensus sequence primed polymerase chain reaction (U.S. Pat. No. 4,437,975), arbitrarily primed polymerase chain reaction (WO90/06995) and nick displacement amplification (WO2004/067726.

In general, the invention provides compositions for use in methods of detecting and/or quantifying a product of a nucleic acid amplification reaction using a dual-labeled primer, herein denoted a FRET primer assay.

In some embodiments, the dual-labeled FRET primer comprises an oligonucleotide, wherein the oligonucleotide is labeled with a fluorophore and a quencher and the oligonucleotide undergoes a detectable change in fluorescence upon extension by at least three nucleotides. In some embodiments, the quencher and fluorophore are separated at a distance such that when the duplex is not polymerized the fluorophore is quenched by the quencher and when the duplex is polymerized the fluorophore is not quenched by the quencher. In some embodiments, the fluorophore and quencher are between about x and y nucleotides apart on the same oligonucleotide. In some embodiments, the distance is between about 4 nucleotides and about 20 nucleotides. In some embodiments, the fluorophore is chosen from fluorescein, 5-carboxyfluorescein (FAM), 2'7'-dimethoxy-4'5'-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), and 5-(2' aminoethyl) amino naphthalene-1-sulfonic acid (EDANS). In some embodiments, the quencher is chosen from: a Black Hole Quencher^{®}, an Iowa Black^{®} quencher, an Eclipse^{®} Dark quencher, a DABCYL quencher and derivatives thereof. In some embodiments, the fluorophore is internal and the quencher is on the 5' end of the oligonucleotide.

In some embodiments, the methods provided herein include the steps of mixing one or more target nucleic acid molecules with one or more fluorescently labeled oligonucleotides. The one or more oligonucleotides are dual-labeled with a fluorophore (fluorescent label) and a quencher and the oligonucleotide undergoes a detectable change in fluorescence upon hybridization of the one or more target nucleic acid molecules. The method includes incubating the mixture with a polymerase under conditions sufficient to synthesize one or more nucleic acid molecules complementary to all or a portion of the one or more target nucleic acid molecules. The one or more synthesized nucleic acid molecules include the one or more oligonucleotides. The method includes detecting the presence or absence or quantifying the amount of the one or more synthesized nucleic acid molecules by measuring the fluorescence.

In other embodiments, the FRET primer assay provided herein includes the steps of:
providing a first and second primer, wherein the first primer is complementary to a sequence within or at or near the 3'-terminus of the first strand of the nucleic acid molecule and the second primer is complementary to a sequence within or at or near the 3'-terminus of the second strand of the nucleic acid molecule;
hybridizing the first primer to the first strand and the second primer to the second strand in the presence of one or more polymerases, under conditions such that the primers are extended to result in the synthesis of a third nucleic acid molecule complementary to all or a portion of the first strand and a fourth nucleic acid molecule complementary to all or a portion of the second strand, denaturing the first and third strands, and the second and fourth strands; and
repeating the above steps one or more times, wherein one of the first and second primers is dual-labeled with a fluorophore and a quencher and wherein the dual-labeled primer undergoes a detectable change in fluorescence upon hybridization of the one or more labeled primers to the nucleic acid molecule. In some embodiments, the change in fluorescence is an increase in fluorescence. In some embodiments, the term "near" includes within 1, 2, 3, 4, 5, 6 or 7 nucleotides of the 3' terminus.

Incubation conditions for the methods disclosed herein may involve the use of one or more nucleotides and one or more nucleic acid synthesis buffers. Such methods may optionally comprise one or more additional steps, such as incubating the synthesized first nucleic acid molecules under conditions sufficient to make one or more second nucleic acid molecules complementary to all or a portion of the first nucleic acid molecules. Such additional steps may also be accomplished in the presence of one or more primers of the present teachings and one or more polymerases as described herein. The invention also relates to nucleic acid molecules synthesized by these methods. Incubation conditions may also involve temperature changes such as those that make conditions ideal for annealing of the primers, denaturing of the templates, denaturing of the newly synthesized nucleic acids, and polymerization by the polymerase.

The methods disclosed herein may be used for detecting the presence of one or more target sequences, quantifying one or more target sequences, and/or identifying the presence of one or more alleles of a target sequence. The target sequence may be any length that is amenable to amplification. The target sequence may be any nucleic acid sequence without exception. The target sequence may include but is not limited to: a viral sequence, a single nucleotide polymorphism (SNP), a bacterial sequence, a sequence identified with a specific disease, highly mutated nucleic acids, small interfering RNAs (siRNAs), and microRNAs (miRNAs). Thus, the methods may be used in methods of diagnosis, pathogen detection, SNP/subtype/mutation detection, gene and RNA detection and/or quantification, and small RNA detection and/or quantification.

The one or more target sequences may be any size that is amenable for amplification. For example, the method is particularly useful for targets that are smaller than those typically used in PCR assays, such as siRNA and miRNA. The methods are also particularly useful for highly mutated nucleic acids such as RNA viral genes. The methods are also particularly useful for fragmented and/or degraded targets or samples, such as forensic samples or fixed tissues.

In an embodiment, each of the steps of the methods are distinct steps. In another embodiment, the steps may not be distinct steps, but may be performed simultaneously. In other embodiments, the methods may not have all of the above steps and/or may have other steps in addition to or instead of those listed above. The steps of the methods may be performed in another order.

### Fluorescent label

Any fluorescent label (fluorophore) may be used without limitation in the methods and compositions disclosed herein. In some embodiments, the fluorophore may be quenched by a known quencher. In some embodiments, the fluorophore may be easily incorporated internally to an oligonucleotide or may be incorporated at or near the 5' end of an oligonucleotide primer. The fluorophore may be on the forward or the reverse primer as long as it is on the same primer as the quencher.

In some embodiments, the fluorophore is a commonly used fluorophore. Fluorophores that are commonly used in FRET include, but are not limited to, fluorescein, 5-carboxyfluorescein (FAM), 2'7'-dimethoxy-4'5'-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), and 5-(2'-aminoethyl)aminonaphthalene-1-sulfonic acid (EDANS). The fluorophore can be any fluorescent label known in the art, including, but not limited to: FAM, TET, HEX, Cy3, TMR, ROX, Texas Red^{®}, LC red 640, Cy5, and LC red 705.

Fluorophores for use in the dual-labeled primer may be chosen from, for example: 4-acetamido-4'-isothiocyanatostilbene-2,2'disulfonic acid; acridine and derivatives (e.g., acridine, acridine isothiocyanate); 5-(2'-aminoethyl)aminonaphthalene1-sulfonic acid (EDANS); 4-amino-N-[3-vinylsulfonyl)phenyl]naphthalimide-3,5 disulfonate (Lucifer Yellow VS); N-(4-anilino-1-naphthyl)maleimide; anthranilamide; Brilliant Yellow; coumarin and derivatives (e.g., coumarin, 7-amino-4-methylcoumarin, 7-amino-4-trifluoromethylcoumarin); cyanosine; 4',6-diaminoidino-2-phenylindole (DAPI); 5',5"-dibromopyrogallol-sulfonephthalein (Bromopyrogallol Red); 7-diethylamino-3-(4'-isothiocyanatophenyl)-4-methylcoumarin; diethylenetraimine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansyl chloride); 4-(4'-dimethylaminophenylazo)benzoic acid (DABCYL); 4-dimethylaminophenylazophenyl-4'-isothiocyanate (DABITC); eosin and derivatives (e.g., eosin, eosin isothiocyanate); erythrosine and derivatives (e.g., erythrosine B, erythrosine isothiocyanate); ethidium; fluorescein and derivatives (e.g., 5-carboxyfluorescein (FAM), 5-(4,6-dichlorotriazin-2-yl)aminofluorescein (DTAF), 2'7'-dimethoxy-4'5'-dichloro-6-carboxyfluorescein (JOE), fluorescein, fluorescein isothiocyanate, and QFITC (XRITC); fluorescamine; IR144; IR1446; Malachite Green isothiocyanate; 4-methylumbelliferone; ortho cresolphthalein; nitrotyrosine;pararosaniline; Phenol Red; B-phycoerythrin; o-phthaldialdehyde; pyrene and derivatives (e.g., pyrene, pyrene butyrate, succinimidyl 1-pyrene butyrate); Reactive Red 4 (Cibacron Brilliant Red 3B-A); rhodamine and derivatives (e.g., 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, sulforhodamine B, sulforhodamine 101, sulfonyl chloride derivative of sulforhodamine 101 (Texas Red^{®}); N,N,N',N'-tetramethyl-6-carboxyrhodamine (TAMRA); tetramethyl rhodamine (tetramethyl rhodamine isothiocyanate (TRITC)); riboflavin; rosolic acid; and terbium chelate derivatives.

Fluorophores for use in the methods disclosed herein may be obtained commercially, for example, from Biosearch Technologies (Novato, CA.), Life Technologies (Carlsbad, CA), GE Healthcare (Piscataway NJ), Integrated DNA Technologies (Coralville, Iowa) and Roche Applied Science (Indianapolis, IN). In some embodiments, the fluorophore is chosen to be usable with a specific detector, such as a specific spectrophotometric thermal cycler, depending on the light source of the instrument. In some embodiments, the fluorophore is chosen to work well with a specific quencher. In some embodiments, if the assay is designed for the detection of two or more target sequences (multiplex amplification assays), and therefore two or more fluorescent hybridization primers may be used, the fluorophores are chosen with absorption and emission wavelengths that are well separated from each other (have minimal spectral overlap).

The fluorophore may be on either primer internally, near the 5' end or at the 5' end as long as the fluorophore and the quencher are situated on the same primer. The fluorophore may be situated on any part of the primer as long as it does not interfere with amplification. The specific part of the primer that the fluorophore is on is not as important as the distance between the fluorophore and quencher. In some embodiments, the fluorophore is situated at distance from the quencher such that when the duplex is not polymerized the fluorophore is quenched by the quencher and when the duplex is polymerized the fluorophore is not quenched by the quencher. Thus, the quencher-fluorophore pair is chosen so that the fluorophore is quenchable by the quencher. The distance may be different for different fluorophore-quencher pairs. For example, the distance may be between about 3 and 30 nucleotides, including between about 4 and 20 nucleotides. In some embodiments, the distance is between about 4 and 14 nucleotides, including 5, 6, 7, 8, 9, 10, 11, 12, and 13 nucleotides. In some embodiments, when the quencher is DABCYL the distance may be about 5 nucleotides.

The quencher may be on the forward or reverse primer as long as it is on the same primer as the fluorophore. Any quencher may be used as long as it decreases the fluorescence intensity of the fluorophore that is being used. Quenchers commonly used for FRET include, but are not limited to, Deep Dark Quencher DDQ-I, Dabcyl, Eclipse^{®}, Iowa Black^{®} FQ, Black Hole Quenchers^{®}, BHQ-1, QSY-7, BHQ-2, DDQ-II, Iowa Black^{®} RQ, QSY-21, and Black Hole Quencher^{®} BHQ-3. Quenchers for use in the methods disclosed herein may be obtained commercially, for example, from Eurogentec (Belgium), Epoch Biosciences (Bothell, WA), Biosearch Technologies (Novato CA), Integrated DNA Technologies (Coralville, Iowa) and Life Technologies (Carlsbad, CA).

The quencher may be situated on any part of the primer as long as it does not interfere with amplification. The quencher may be on either primer, internally, near the 5' end or at the 5' end as long as the fluorophore and the quencher are situated on the same primer. The specific region of the primer that the quencher is on is not as important as the distance between the fluorophore and quencher.

The quencher can be situated at distance from the fluorophore such that when the duplex is not polymerized the fluorophore is quenched by the quencher and when the duplex is polymerized the fluorophore is not quenched by the quencher. Thus, the quencher-fluorophore pair is chosen so that the fluorophore is quenchable by the quencher. The distance can be different for different fluorophore-quencher pairs. For example, the distance can be between about 3 and 30 nucleotides, including between about 4 and 20 nucleotides. In some embodiments, the distance is between about 4 and 14 nucleotides, including about 5, 6, 7, 8, 9, 10, 11, 12, and 13 nucleotides. In some embodiments, when the quencher is DABCYL the distance may be about 5 nucleotides.

### Dual-labeled Primer

The methods and compositions of the FRET primer assay disclosed herein provide oligonucleotides for nucleic acid amplification that are incorporated into the amplified product and that utilize the principle of fluorescence resonance energy transfer (FRET). The oligonucleotides include a forward and a reverse primer, wherein one of the primers is a dual-labeled oligonucleotide. The dual-labeled oligonucleotide is labeled with both a fluorophore and a quencher. The fluorophore (fluorescent labeling moiety) and/or the quencher on the oligonucleotide primer are not situated so as to substantially interfere with subsequent ligation at its 3' end to the selected primer sequence. Thus, a labeling moiety (a quencher or a fluorophore) is not located on the 3' terminal nucleotide of the oligonucleotide primer. The fluorophore may be internal or 5' terminal. The quencher may be internal or 5' terminal. However, when producing the oligonucleotide in some cases it will be advantageous for the quencher to be attached at the end of the template and the fluorophore to be attached internally. This is because with currently available methods, it is easier to attach the fluorophore within the oligonucleotide while it is being produced. However, it is envisioned that new methods may make it advantageous to incorporate the quencher into the oligonucleotide as it is being produced.

The fluorescent and quencher moieties may be separated by a distance such that when the duplex is not polymerized, the emissions of the fluorophore are quenched by the quencher. This may be easily determined by one of ordinary skill in the art using techniques known in the art. In some embodiments, the fluorophore and quencher are separated by a distance that still gives fluorescence, but is not so far that the background is overly high. For example, when testing the quencher BHQ it was found that when the fluorophore and the BHQ were separated by 3 nucleotides the fluorophore did not fluoresce at all when polymerized. Further, when the fluorophore and BHQ were separated by 14 nucleotides the background was too high. Thus, the fluorophore and quencher are separated by a distance of between about 3 and 30 nucleotides, including, but not limited to, about 4 and 20 nucleotides, including about 5, 6, 7, 8, 9, 10, 11, 12, 13, 14, 15, 16, 17, 18, and 19 nucleotides. In some embodiments, the distance between the fluorophore and quencher depends upon the quencher used and may depend upon the specific quencher-fluorphore pair used. In some embodiments, when the quencher is DABCYL, the distance is about 5 nucleotides. In some embodiments, the two FRET moieties (fluorophore and quencher) are separated by an intervening sequence long enough provide a distance of between about 3 and 30 nucleotides between a fluorophore and a quencher when the primer is not polymerized. In some embodiments, when the quencher is located on the 5' end, the fluorophore is located between about 1 and 6 nucleotides from the 3' end, including but not limited to about 2 nucleotides, 3 nucleotides, 4 nucleotides and 5 nucleotides.

The skilled artisan can determine, using art-known techniques of spectrophotometry, which fluorophore and quencher pair will make a suitable FRET pair. For example, in some embodiments, fluoroscein and Iowa Black^{®} FQ or FAM and BHQ1 are used for a FRET primer. In some embodiments, when FAM and BHQ1 are used the distance between the FRET pair is between about 3 and 20 nucleotides (nt). In some embodiments, the distance is between about 5 and 19 nucleotides, including about 6, 7, 8, 9, 10, 11, 12, 13, 14, 15, 16, 17 and 18 nucleotides.

The primers (oligonucleotides) for use in the amplification reactions disclosed herein may be any suitable size, including but not limited to, in the range of 10-100 nucleotides or 10-80 nucleotides, or 20-40 nucleotides.

The primers (oligonucleotides) may be DNA or RNA or chimeric mixtures or derivatives or modified versions thereof, so long as they are still capable of priming the desired amplification reaction. In addition to being labeled with a fluorophore and quencher, the oligonucleotide may be modified at the base moiety, sugar moiety, or phosphate backbone, and may include other appending groups or labels, so long as it is still capable of priming the desired amplification reaction.

For example, the primer (oligonucleotide) may comprise at least one modified base moiety which is selected from the group including but not limited to 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from the group including but not limited to arabinose, 2-fluoroarabinose, xylulose, and hexose.

In yet another embodiment, the oligonucleotide comprises at least one modified phosphate backbone selected from the group including but not limited to a phosphorothioate, a phosphorodithioate, a phosphoramidothioate, a phosphoramidate, a phosphordiamidate, a methylphosphonate, an alkyl phosphotriester, and a formacetal or analog thereof.
In some embodiments, the oligonucleotides may be modified to more strongly bind to the target. Examples of modifications that may enhance the binding or an RNA or DNA or to its target include but are not limited to: 2'-O-alkyl modified ribonucleotides, 2'-O-methyl ribonucleotides, 2'-orthoester modifications (including but not limited to 2'-bis(hydroxyl ethyl), and 2' halogen modifications and locked nucleic acids (LNAs).

In some embodiments, methods for synthesizing oligonucleotides are conducted using an automated DNA synthesizer by methods known in the art. As examples, phosphorothioate oligonucleotides may be synthesized by the method of Stein et al. (1988, Nucl. Acids Res. 16:3209-3221), methylphosphonate oligonucleotides may be prepared by use of controlled pore glass polymer supports (Sarin et al., 1988, Proc. Natl. Acad. Sci. U.S.A. 85:7448-7451), etc. Once the desired oligonucleotide is synthesized, it is cleaved from the solid support on which it was synthesized and treated, by methods known in the art, to remove any protecting groups present. The oligonucleotide may then be purified by any method known in the art, including extraction and gel purification. The concentration and purity of the oligonucleotide may be determined by examining the oligonucleotide that has been separated on an acrylamide gel, or by measuring the optical density at 260 nm in a spectrophotometer. The oligonucleotides disclosed herein may be derived by standard phosphoramidite chemistry, or by cleavage of a larger nucleic acid fragment using non-specific nucleic acid cleaving chemicals or enzymes or site-specific restriction endonucleases.

Oligonucleotides of the present teachings may be labeled with fluorophore and quencher moieties during chemical synthesis or the label may be attached after synthesis by methods known in the art. In general, labeling methods well known in the art may involve the use of, for example, RNA ligase, polyA polymerase, terminal transferase, or by labeling the RNA backbone, etc.; see, e.g., Ausubel, et al., Short Protocols in Molecular Biology, 3rd ed., Wiley & Sons 1995 and Sambrook et al., Molecular Cloning: A Laboratory Manual, Third Edition, 2001 Cold Spring Harbor, N.Y.

### Targets

The targets of the invention may be any nucleic acid target known to the skilled artisan. Further, the targets may be regions of low mutation or regions of high mutation. For example, one particularly valuable use of the methods disclosed herein involves targeting highly mutated nucleic acids, such as RNA viral genes. In some embodiments, the targets may be fragmented or degraded, such as material from forensic samples and/or fixed tissues.

The targets may be any size amenable to amplification. The targets may be chosen from a wide variety of sizes. For example, the targets may be long fragments or short fragments. One particularly valuable use of the methods and compositions provided herein involves the identification of short fragments, such as siRNA and miRNA. Another particularly valuable use is for samples that may have fragmented and/or degraded nucleic acid, such as fixed samples or samples that have been exposed to the environment. Thus, the methods may be used for biopsy tissue, and forensic DNA for example.

The targets may be purified or unpurified. The targets may be produced (for example cDNA) or can be found in biological samples. The biological sample may be used without treatment or the biological samples may be treated to remove substances that may interfere with the methods disclosed herein.

The FRET primers provided herein may be used in methods of diagnosis, whereby the primers are complementary to a sequence (e.g., genomic) of an infectious disease agent, e.g., of human disease including but not limited to viruses, bacteria, parasites, and fungi, thereby diagnosing the presence of the infectious agent in a sample having nucleic acid from a patient. The target nucleic acid may be genomic or cDNA or mRNA or synthetic, human or animal, or of a microorganisms, etc. In other embodiments, the primers may be used to diagnose or prognose a disease or disorder that is not caused by an infectious agent. For example, the primers may be used to diagnose or prognose cancer, autoimmune diseases, mental illness, genetic disorders, etc. by identifying the presence of a mutation, polymorphism, or allele in a sample from a human or animal. In some embodiments, the primer comprises the mutation or polymorphism. In some embodiments, different sets of primers amplify respectively, the wild type sequence or the mutated version.

The FRET dual-labeled primers disclosed herein may be used in methods that may include targets that have been fragmented or degraded. For example, one valuable use for the dual-labeled primers is FFPE (formalin-fixed paraffin embedded tissue). This is because the treatment of the tissue can often lead to fragmentation and/or degradation of the nucleic acid. However, the methods using the FRET dual-labeled primers can be performed on very small fragments (e.g., degraded nucleic acids).

### Polymerases

As used herein "polymerase" refers to any enzyme having a nucleotide polymerizing activity. Any polymerase amenable to amplifying a target can be used in the methods provided herein, including polymerases that do not have exonuclease and/or endonuclease activity. Thus, unlike some methods, the methods using the FRET dual-labeled primers do not require that the enzyme have exonuclease activity.

Polymerases (including DNA polymerases and RNA polymerases) useful in accordance with the present invention include, but are not limited to, *Thermus thermophilus* (Tth) DNA polymerase, *Thermos aquaticus* (Taq) DNA polymerase, *Thermotoga neopolitana* (Tne) DNA polymerase, *Thermotoga maritima* (Tma) DNA polymerase, *Thermococcus litoralis* (Tli or VENT™) DNA polymerase, *Pyrococcus furiosus* (Pfu) DNA polymerase, DEEPVENT™ DNA polymerase, *Pyrococcus woosii* (Pwo) DNA polymerase, *Bacillus sterothermophilus* (Bst) DNA polymerase, *Bacillus caldophilus* (Bca) DNA polymerase, *Sulfobus acidocaldarius* (Sac) DNA polymerase, *Thermoplasma acidophilum* (Tac) DNA polymerase, *Thermus flavus* (Tfl/Tub) DNA polymerase, *Thermus ruber* (Tru) DNA polymerase, *Therms brockianus* (DYNAZYME™) DNA polymerase, *Methanobacterium thermoautotrophicum* (Mth) DNA polymerase, mycobacterium DNA polymerase (Mtb, Mlep), and mutants, and variants and derivatives thereof. RNA polymerases such as T3, T5 and SP6 and mutants, variants and derivatives thereof may also be used in accordance with the invention. Generally, any type I DNA polymerase may be used in accordance with the invention although other DNA polymerases may be used including, but not limited to, type III or family A, B, C etc. DNA polymerases.

Polymerases used in accordance with the invention may be any enzyme that can synthesize a nucleic acid molecule from a nucleic acid template, typically in the 5' to 3' direction. The nucleic acid polymerases used in the methods disclosed herein may be mesophilic or thermophilic. Exemplary mesophilic DNA polymerases include T7 DNA polymerase, T5 DNA polymerase, Klenow fragment DNA polymerase, DNA polymerase III and the like. Exemplary thermostable DNA polymerases that may be used in the methods of the invention include Taq, Tne, Tma, Pfu, Tfl, Tth, Stoffel fragment, VENT™ and DEEPVENT™ DNA polymerases, and mutants, variants and derivatives thereof (U.S. Pat. No. 5,436,149; U.S. Pat. No. 4,889,818; U.S. Pat. No. 4,965,188; U.S. Pat. No. 5,079,352; U.S. Pat. No. 5,614,365; U.S. Pat. No. 5,374,553; U.S. Pat. No. 5,270,179; U.S. Pat. No. 5,047,342; U.S. Pat. No. 5,512,462; WO 92/06188; WO 92/06200; WO 96/10640; Barnes, W. M., Gene 112:29-35 (1992); Lawyer, F. C., et al., PCR Meth. Appl. 2:275-287 (1993); Flaman, J.-M, et al., Nucl. Acids Res. 22(15):3259-3260 (1994)). Examples of DNA polymerases substantially lacking in 3' exonuclease activity include, but are not limited to, Taq, Tne(exo-), Tma(exo-), Pfu (exo-), Pwo(exo-) and Tth DNA polymerases, and mutants, variants and derivatives thereof.

DNA polymerases for use in the methods disclosed herein may be obtained commercially, for example, from Life Technologies, Inc. (Rockville, Md.), Pharmacia (Piscataway, N.J.), Sigma (St. Louis, Mo.) and Boehringer Mannheim. Exemplary commercially available DNA polymerases for use in the present invention include, but are not limited to, Tsp DNA polymerase from Life Technologies, Inc.

Enzymes for use in the compositions, methods, compositions and kits provided herein include any enzyme having reverse transcriptase activity. Such enzymes include, but are not limited to, retroviral reverse transcriptase, retrotransposon reverse transcriptase, hepatitis B reverse transcriptase, cauliflower mosaic virus reverse transcriptase, bacterial reverse transcriptase, Tth DNA polymerase, Taq DNA polymerase (Saiki, R. K., et al., Science 239:487-491 (1988); U.S. Pat. Nos. 4,889,818 and 4,965,188), Tne DNA polymerase (WO 96/10640), Tma DNA polymerase (U.S. Pat. No. 5,374,553) and mutants, fragments, variants or derivatives thereof (see, e.g., commonly owned, co-pending U.S. patent application Ser. Nos. 08/706,702 and 08/706,706, both filed Sep. 9, 1996). As will be understood by one of ordinary skill in the art, modified reverse transcriptases and DNA polymerase having RT activity may be obtained by recombinant or genetic engineering techniques that are well-known in the art. Mutant reverse transcriptases or polymerases may, for example, be obtained by mutating the gene or genes encoding the reverse transcriptase or polymerase of interest by site-directed or random mutagenesis. Such mutations may include point mutations, deletion mutations and insertional mutations. In some embodiments, one or more point mutations (e.g., substitution of one or more amino acids with one or more different amino acids) are used to construct mutant reverse transcriptases or polymerases for use in the invention. Fragments of reverse transcriptases or polymerases may also be obtained by deletion mutation by recombinant techniques that are well-known in the art, or by enzymatic digestion of the reverse transcriptase(s) or polymerase(s) of interest using any of a number of well-known proteolytic enzymes.

In some embodiments, enzymes for use in the methods provided herein include those that are reduced or substantially reduced in RNase H activity. Such enzymes that are reduced or substantially reduced in RNase H activity may be obtained by mutating the RNase H domain within the reverse transcriptase of interest, for example, by one or more point mutations, one or more deletion mutations, or one or more insertion mutations as described above. An enzyme "substantially reduced in RNase H activity" refers to an enzyme that has less than about 30%, less than about 25%, less than about 20%, less than about 15%, less than about 10%, less than about 7.5%, or less than about 5%, or less than about 5% or less than about 2%, of the RNase H activity of the corresponding wild type or RNase H⁺ enzyme such as wild type Moloney Murine Leukemia Virus (M-MLV), Avian Myeloblastosis Virus (AMV) or Rous Sarcoma Virus (RSV) reverse transcriptases. The RNase H activity of any enzyme may be determined by a variety of assays, such as those described, for example, in U.S. Pat. No. 5,244,797, in Kotewicz, M. L., et al., Nucl. Acids Res. 16:265 (1988), in Gerard, G. F., et al., FOCUS 14(5):91 (1992), and in U.S. Pat. No. 5,668,005.

Polypeptides having reverse transcriptase activity for use in the methods provided herein may be obtained commercially, for example, from Life Technologies, Inc. (Rockville, Md.), Pharmacia (Piscataway, N.J.), Sigma (Saint Louis, Mo.) or Boehringer Mannheim Biochemicals (Indianapolis, Ind.). Alternatively, polypeptides having reverse transcriptase activity may be isolated from their natural viral or bacterial sources according to standard procedures for isolating and purifying natural proteins that are well-known to one of ordinary skill in the art (see, e.g., Houts, G. E., et al., J. Virol. 29:517 (1979)). In addition, the polypeptides having reverse transcriptase activity may be prepared by recombinant DNA techniques that are familiar to one of ordinary skill in the art (see, e.g., Kotewicz, M. L., et al., Nucl. Acids Res. 16:265 (1988); Soltis, D. A., and Skalka, A. M., Proc. Natl. Acad. Sci. USA 85:3372-3376 (1988)).

Exemplary polypeptides having reverse transcriptase activity for use in the methods provided herein include M-MLV reverse transcriptase, RSV reverse transcriptase, AMV reverse transcriptase, Rous Associated Virus (RAV) reverse transcriptase, Myeloblastosis Associated Virus (MAV) reverse transcriptase and Human Immunodeficiency Virus (HIV) reverse transcriptase, and others described in WO 98/47921 and derivatives, variants, fragments or mutants thereof, and combinations thereof. In a further embodiment, the reverse transcriptases are reduced or substantially reduced in RNase H activity, and may be selected from the group consisting of M-MLV H- reverse transcriptase, RSV H- reverse transcriptase, AMV H- reverse transcriptase, RAV H- reverse transcriptase, MAV H- reverse transcriptase and HIV H- reverse transcriptase, and derivatives, variants, fragments or mutants thereof, and combinations thereof. Reverse transcriptases of particular interest include AMV RT and M-MLV RT, and optionally AMV RT and M-MLV RT having reduced or substantially reduced RNase H activity (e.g., AMV RT alpha H-/BH+ and M-MLV RT H-). Reverse transcriptases for use in the invention include SuperScript™, SuperScript™II, ThermoScript™ and ThermoScript™ II available from Life Technologies, Inc. See generally, WO 98/47921, U.S. Pat. Nos. 5,244,797 and 5,668,005.

### Detection

The detection of the signal may be using any reagents or instruments that detect a change in fluorescence from a fluorophore. For example, detection may be performed using any spectrophotometric thermal cycler. Examples of spectrophotometric thermal cyclers include, but are not limited to, Applied Biosystems (AB) PRISM^{®} 7000, AB 7300 real-time PCR system, AB 7500 real-time PCR system, AB PRISM^{®} 7900HT, Bio-Rad ICycler IQ™, Cepheid SmartCycler^{®} II, Corbett Research Rotor-Gene 3000, Idaho Technologies R.A.P.I.D.™, MJ Research Chromo 4™, Roche Applied Science LightCycler^{®}, Roche Applied Science LightCycler^{®}2.0, Stratagene Mx3000P™, and Stratagene Mx4000™. It should be noted that new instruments are being developed at a rapid rate and any like instruments may be used for the methods.

### Kits

Some embodiments of the present teachings provide kits for the quantification or detection of one or more target nucleic acid molecules in a sample during nucleic acid synthesis, including a dual-labeled oligonucleotide. The dual-labeled oligonucleotide or primer includes a fluorophore at one location and a quencher at another location, such that the quencher and fluorophore are separated at a distance that when the duplex is not polymerized the fluorophore is quenched by the quencher and when polymerized the fluorophore is not quenched by the quencher. The kit may be used in methods of polymerase chain reaction. Multiplexing refers to the determination of expression of multiple genes in a single sample. In some embodiments the kit is used for multiplexed reactions, such as to identify one or more alleles or single nucleotide polymorphisms in a sample.

### Samples

The methods and compositions may be used for detection and quantification of nucleic acids in a sample. The sample may include one or more templates and/or one or more target nucleic acids. The sample may be purified or unpurified. The sample may be a biological sample, such as blood, saliva, tears, tissue, urine, stool, etc., that has been treated to use in the methods provided herein. Alternatively, if the biological sample does not interfere with the methods provided herein, it may be used untreated (or unpurified).

### EXAMPLES

The following examples provide methods and compositions for FRET primer assays provided herein. The FRET primer assay methods disclosed herein were partially based on the discovery that when a dual-labeled primer labeled with a fluorophore and a quencher (one internal and the other 5' terminal) was used, the extension by a DNA polymerase by at least 3 nucleotides released fluorescence. Without being bound by a specific theory, it is thought that the extension forces the fluor-quench pair apart by the rigidity of the double-stranded structure. The increase in fluorescence increased with the amount of extended primer in a direct relationship, providing a new method for quantifying DNA amplification. Furthermore, it was discovered that when the FRET primers were designed such that the quencher and fluorophore were separated from each other at a distance such that when the duplex is not polymerized the fluorophore is quenched by the quencher and when the duplex is polymerized the fluorophore is not quenched by the quencher, there is no need for additional treatments to separate or remove the unincorporated primer from the amplification product to remove background fluorescence.

The FRET primer assay provided herein obviates the need for an additional fluorogenic probe that anneals to a sequence between the pair of primers as routinely used in other PCR methods (i.e. Taqman^{®}), enabling the use of very small targets. This lends itself to the detection of small targets (i.e. miRNA, siRNA), or targets with very small discriminating regions (i.e. RNA viruses or SNPs). Further, because there is no probe requirement, it enables faster PCR, the limit is just the scanning speed of the PCR machine used. When dissociated, the fluorescence from the single-stranded extended FRET primers is again quenched, producing a melting curve. By this means, it provides a method of surveillance for the specificity of the amplifications and assists in making plus or minus calls on samples with very high Cₜs. When compared to LUX, the FRET primer assays taught herein do not need a hairpin structure in the primers allowing for primers to be 100% homologous to the target and allowing for easy design and more efficient amplification. In the same manner as the SNPLex assays, with differently-labelled allele specific primers, the FRET primer assays taught herein can differentiate between specific SNPs in one tube reactions.

The following methods were used for all of the experiments detailed below in the Examples except as otherwise noted.

All reagents, unless specifically mentioned, were obtained from Life Technologies Inc. AmpliTaq Gold^{®} DNA Polymerase ; AmpliTaq^{®} DNA Polymerase, Stoffel Fragment; AB 7500.

TaqMan^{®} primers and probes were synthesized by Life Technologies Inc, the FRET labeled reverse primer was synthesized by IDT inc., FAM was labeled with an internal Fluorescein dT, the quencher at the 5' end was 5' Iowa Black^{®} FQ. Three labeled primers were used in the experiments, the first primer (Primer 1) was 3' blocked by FAM-labeled dT and thus did not amplify (negative control), the third primer (Primer 3) had only 3 bases between the FAM and the quencher, so while it might produce amplification, there would be no signal, only the second primer (Primer 2) with 14 bases yielded a signal.
Primer 1: quencher-GGTCACCCACCTCGAACGT (SEQ ID NO:4);
Primer 2: quencher-GGTCACCCACCTCGAACGT (SEQ ID NO:5);
Primer 3: quencher-GGTCACCCACCTCGAACGT (underlined T is FAM-labeled) (SEQ ID NO:6).

Synthetic equine herpes virus (EHV1) DNA were synthesized by IDT inc. and were serially diluted and used as PCR amplification and detection targets. PCR reactions were carried out in an AB7500 Fast® PCR machine using a standard ramping speed, 95°C 1 min; [95°C 15 sec, 60 °C 1min] x40 cycles. The PCR volume was 25 µl in AmpliTaq Gold^{®} complete PCR buffer, with 5 U of each Taq enzyme for each reaction; the final concentration of dNTPs was 0.4 mM; the PCR primers and TaqMan^{®} probe final concentration was at 0.9 µM and 0.25 µM respectively.

### Example 1: FRET primer assays - non-hydrolysis based probeless assays based on the extension and duplex formation of a dual-labeled primer.

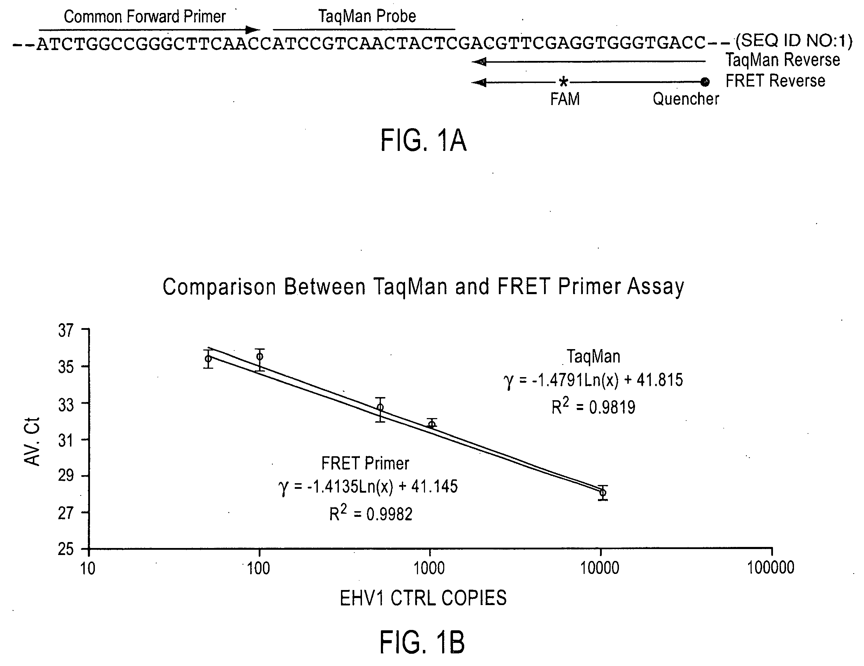

The equine herpes virus 1 (EHV1) polymerase gene was used as a PCR amplification and detection template to compare the FRET primer assay disclosed herein with the TaqMan^{®} assay. Real-time quantification of serially diluted EHV-1 DNA targets was performed. FRET primers were used with only the reverse primer being dual-labeled with the Fluorophore FAM and the quencher BHQ1(see Figure 1). PCR's were performed targeting serially diluted EHV-1 DNA targets. Figure 1 provides the sequence of the forward and reverse primers for the FRET and TaqMan^{®} assays.

PCR was performed as in the methods above. Figure 1 shows the EHV 1 DNA template and primer sequences. The EHV 1 DNA template had the following sequence:
ATCTGGCCGGGCTTCAACCATCCGTCAACTACTCGACGTTCGAGGTGGGTGACC (SEQ ID NO:1) The Common forward primer was: ATCTGGCCGGGCTTCAAC (SEQ ID NO:7). The FRET reverse primer was dual-labeled with an internal FAM and a 5' Iowa Black FQ quencher and had the sequence: TGATGCAGTGCAAGCTCCACCCACTGG (SEQ ID NO:8). The TaqMan^{®} reverse primer had the same sequence without the labels. The TaqMan^{®} probe had the sequence ATCCGTCAACTACTC (SEQ ID NO:9) internal to the forward and reverse primers.

The results of this experiment indicated that FRET PCR without a probe linearly detected serially diluted EHV-1 DNA as well as the TaqMan^{®} assay (Figure 1). The FRET primer displayed similar quantification over a range of 50 to 10,000 copies of EHV 1 template DNA as compared to the TaqMan^{®} assay. The results showed that dual labeled oligos with free 3' termini could be used for real time quantitative PCR.

### Example 2: Dependence of the FRET primer assay on hydrolysis

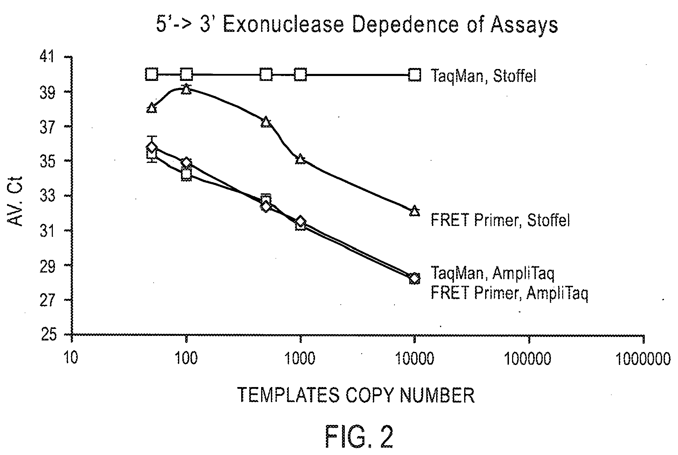

The TaqMan^{®} assay relies on the hydrolysis of a dual labeled probe (5' reporter dye and 3' quencher dye) by Taq Polymerase's 5' → 3' exonuclease activity. The FRET primer assay was compared to the TaqMan^{®} assay in Figure 2 using the Stoffel enzyme. The Stoffel enzyme is a Taq polymerase without 5'→3' exonuclease activity. PCR was performed as in Example 1 using the EHV-1 target DNA and the primers shown in Figure 1.

As shown in Figure 2, when the Stoffel enzyme was used, the TaqMan^{®} assay was incapable of detecting EHV1 DNA, while the FRET primer assay was able to produce a fluorescence signal and to detect the EHV-1 DNA target. Thus, the FRET primer assay did not rely on the 5'→ 3' exonuclease activity of Taq enzyme. The FRET primer assay using Stoffel Taq polymerase provided equivalent quantification of serially diluted EHV-1 DNA target as the FRET primer assay using the Taq enzyme. In contrast, the TaqMan^{®} assay using the Stoffel Taq polymerase was completely incapable of detecting the EHV-1 target.

### Example 3: FRET primer assay functionality involves duplex formation of the labeled amplified product.

Dissociation curve analysis was performed on the amplified PCR products from Example 2. Dissociation analyses were performed on the Applied Biosystems 7500FAST PCR machine (Life Technolgies, Foster City, CA) to determine the melting temperature (Tₘ) of nucleic acid target sequences in samples. The samples were gradually heated from 60°C to 95°C, and the fluorescence signals were collected. The results of the dissociation experiment were plotted as the derivative data (Rn'), which is the negative of the rate of change in fluorescence as a function of temperature, versus temperature (T). The Tm for the target nucleic acid was visible as the maximum for the rate of change (displayed as a peak) for the appropriate dissociation curve.

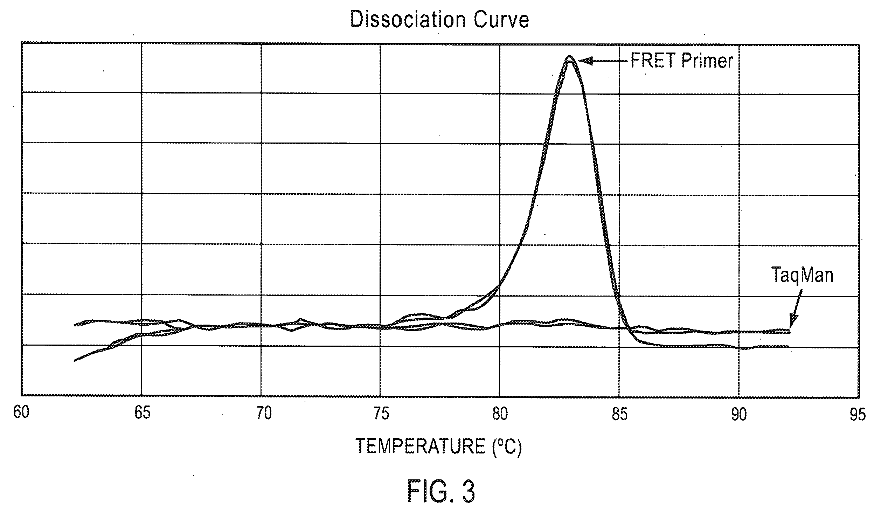

The dissociation analysis (Figure 3) showed that, upon extension/duplex formation, the FRET primer formed a rigid structure which resulted in the unquenched state and produced a fluorescence increase. In the dissociated or single-stranded state (above the Tₘ) the FRET primer or extended product resulted in the random coiled structure and produced the quenched state. This indicated that the formation of a duplex amplified product resulted in the change from the quenched state to fluorescence. The TaqMan^{®} assay did not show this reliance. Thus, FRET primers can provide extended PCR applications in reactions in which the TaqMan^{®} probe is non-functional such as mutation subtype, or SNP detection using high resolution melting curve analysis and multiplex PCR.

### Example 4: FRET primer assay functionality involves the extension of labeled primer.

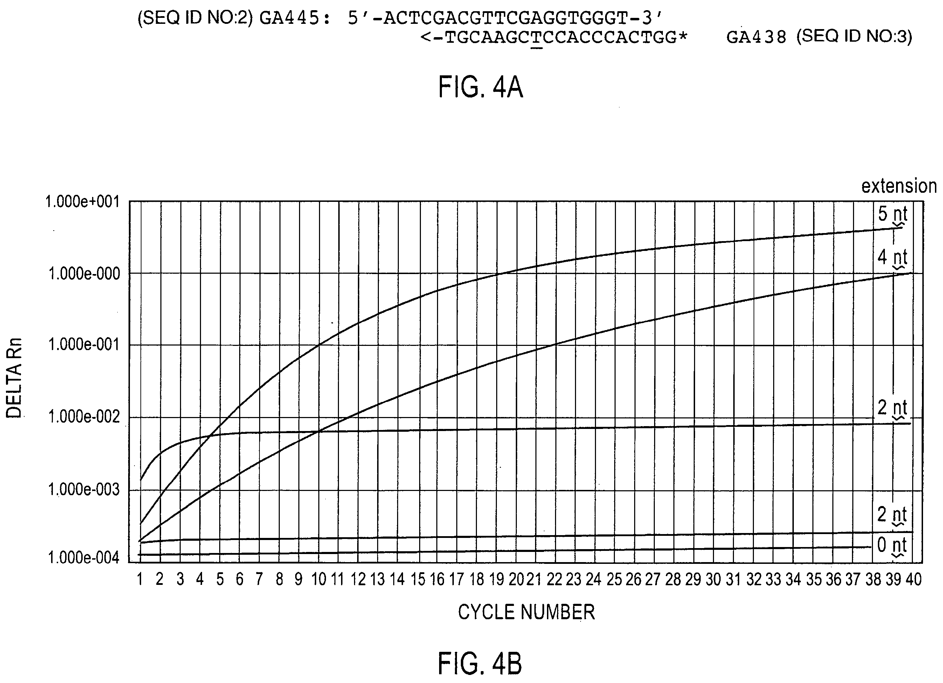

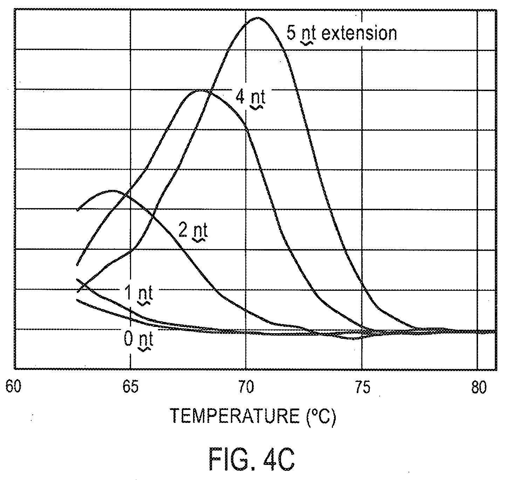

In order to determine if the fluorescence is a result from the annealing of the labeled primer to the target or from extension of the labeled primer, PCR was performed using a reverse complimentary oligo (GA445) and a FRET primer (GA438) (see Figure 4A) in five PCR reactions. The reverse complimentary oligo GA445 had the following sequence: ACTCGACGTTCGAGGTGGGT (SEQ ID NO:2) and GA438 had the following sequence: TGCAAGCTCCACCCACTGG (SEQ ID NO:3). Each reaction contained various numbers of available dNTPs to generate up to a 5 nucleotide extension from the FRET primer. The control had no dNTPs. After 40 cycles of PCR (Figure 4B), the results showed that products with an extension of more than 2 nucleotides from the FRET primer produced significant fluorescence. These products were further subjected to dissociation, and the results (Figure 4C) revealed that extension of more than 2 nucleotides was necessary for producing significant fluorescence peaks in the dissociation assay. Thus, extension from the FRET primers of more than 2 nucleotides was required to produce significant fluorescence release. Products with extension from FRET primers of more than 2 nucleotides produced significant fluorescence during real-time PCR detection and fluorescence peaks during dissociation analysis. Products without extension or only one nucleotide extension from FRET primers did not produce much fluorescence during the same procedures.

### Example 5: The FRET primer assay is different from other PCR assays which rely on secondary structures of primers or probes.

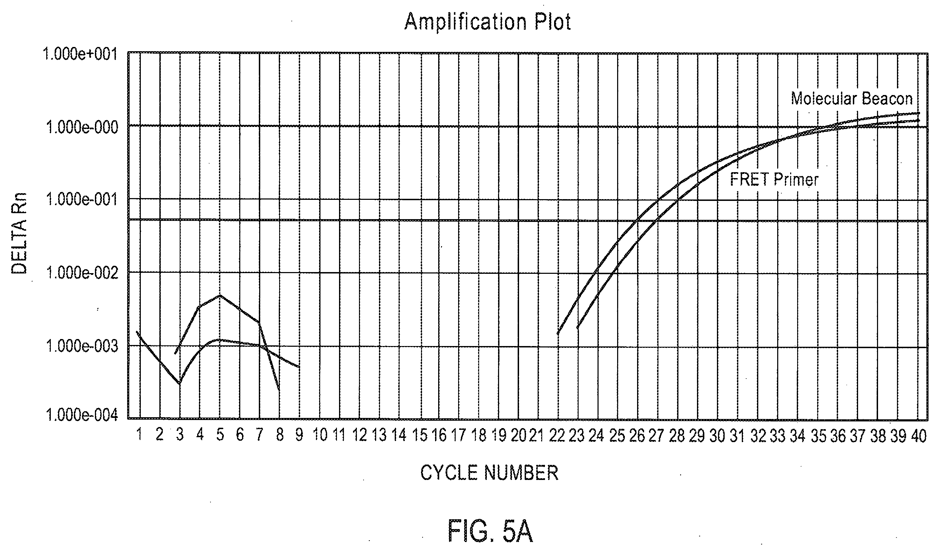

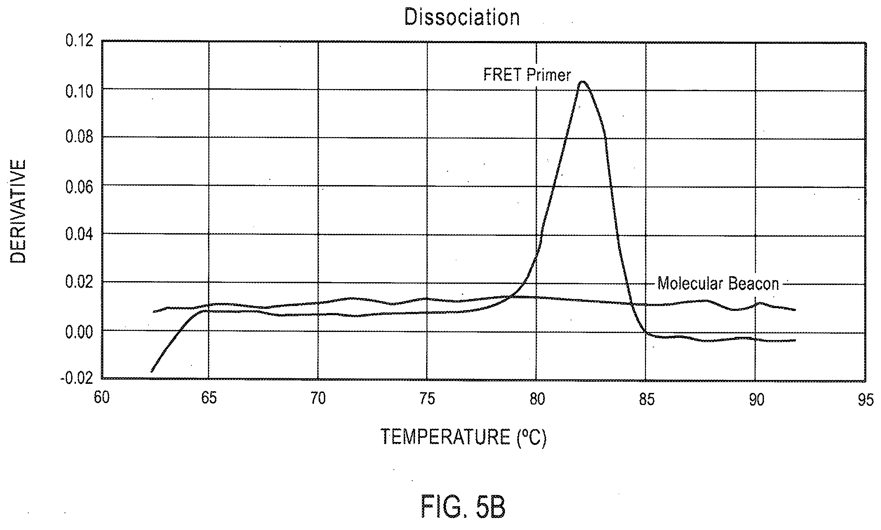

The non-TaqMan^{®} PCR assays were compared to the FRET primer assay and analyzed by melting curve analysis. The primers and probes are provided in Table 1. Most of the non-TaqMan^{®} real-time PCR detection assays utilize secondary structure dependent mechanisms: the fluorophores are separated from quenchers and the fluorescence is released when labeled oligos anneal to target PCR products. In order to demonstrate that the FRET primer assay disclosed herein was different, FRET primer assays and molecular beacon assays were employed to monitor the PCR amplification of EHV1 and Xeno DNA respectively. The two assays displayed similar tracking of PCR amplification of the two reactions (Figure 5A), however, when PCR products were subject to dissociation, the FRET primer assay displayed an abrupt drop in fluorescence intensity at 82°C, indicating the dissociation of duplex DNA structure at this temperature. As shown in Figure 5B, upon reaching 82°C, the extended strand from the FRET primer dissociated from the complimentary strand and the fluorescence was quenched, manifested as a sharp drop in the fluorescence. The fluorescence produced from the molecular beacons however remained relatively steady with increasing temperature. Meanwhile, the increasing temperature dissociated the molecular beacon from its targets, but also prevented its ability to fold back to the hairpin secondary structure. Thus, there was not a sudden decrease in the fluorescence. The difference in the dissociation procedure indicates that the FRET primer assay is different from those that rely on the secondary structure of labeled oligos.

**Table 1: Probes and Primers used in TaqMan^{®} assays:**

| | |
|---|---|
| EHV1 forward | ATCTGGCCGGGCTTCAAC (SEQ ID NO:7) |
| EHV1 FRET reverse | BHQ1-GGTCACCCACC(int-FAM T)CGAACGT (SEQ ID NO:6) |
| Molecular Beacon for Xeno DNA | FAM-cgctc(GTTACTCGTCAGGCACTCGGT)gagcg-BHQ1 (SEQ ID NO:26) |

Thus, the FRET primer assays disclosed herein have several advantages over other probeless assays (such as Sunrise primers, molecular beacons, Scorpions and LUX primers), including simpler primer design and removing the requirement of resuming a specific secondary structure for fluorescence quenching to occur.

### Example 6: Comparison of the FRET primer assay to the SYBR Green^{®} assay for amplicon size differentiation using melting curve analysis.

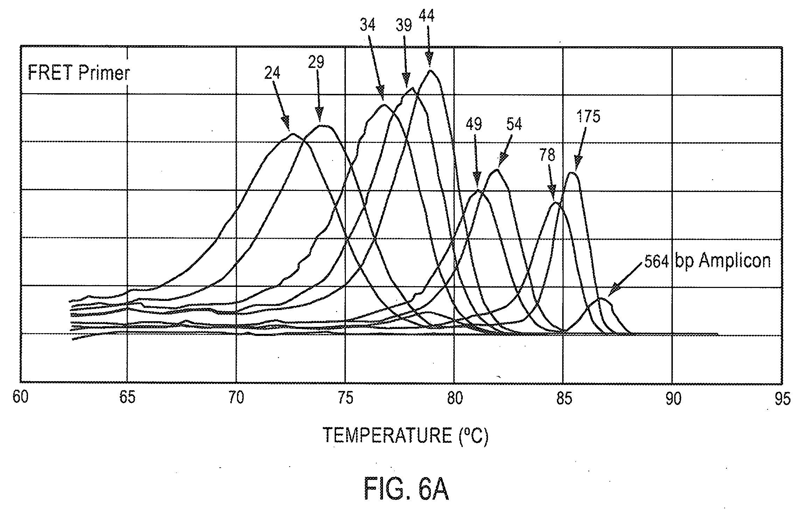

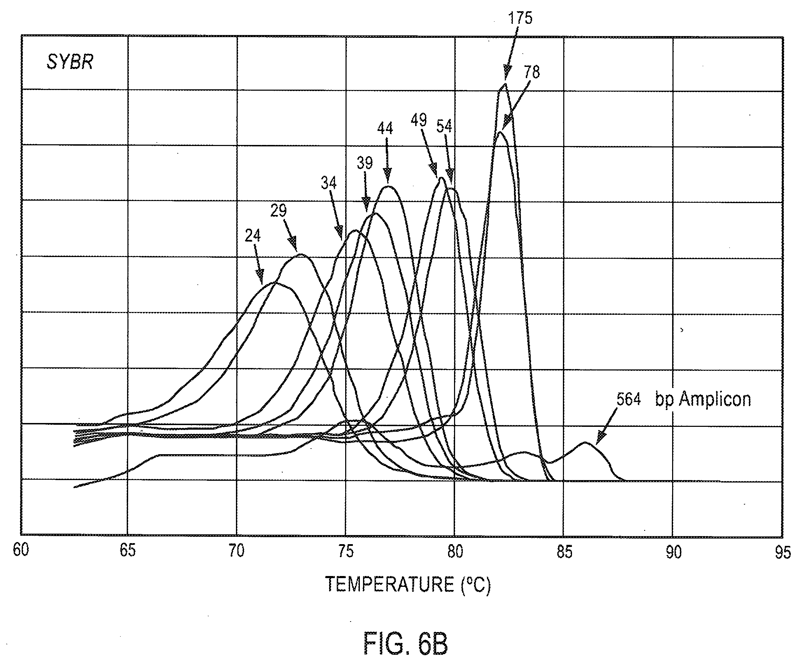

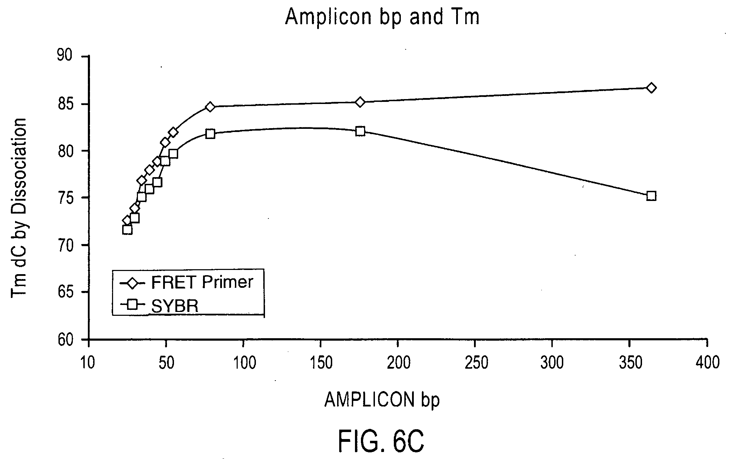

The SYBR Green^{®} assay was compared to the FRET primer assay and analyzed by melting curve analysis (see Figure 6A for FRET primer and 6B for SYBR Green^{®}). The respective primer sets for ten amplicons were used for PCR as shown in Table 2. As shown in Figure 6C, when looking at amplicon size and Tₘ, the FRET primer assays provided better amplicon size differentiation by melting curve analysis than the SYBR Green^{®} assays. Amplicon size differentiation was more resolved with FRET primers.

**Table 2: Primer sets used for PCR**

| amplicon size (bp) | forward primer | common SYBR reverse primer | common reverse FRET primer |
|---|---|---|---|
| 54 | ATCTGGCCGGGCTTCAAC (SEQ ID NO:7) | | |
| 78 | CCACCCTGGCGCTCG (SEQ ID NO:27) | | |
| 49 | GCCGGGCTTCAACCATCC (SEQ ID NO:28) | | |
| 44 | GCTTCAACCATCCGTCAACTACTCGA C (SEQ ID NO:29) | | |
| 39 | AACCATCCGTCAACTACTCGACGTTC (SEQ ID NO:30) | GGTCACC CACCTCG AACGT (SEQ ID NO:4) | BHQ1-GGTCACCCACC( int-FAMT)CGAACGT (SEQ ID NO:6) |
| 34 | TCCGTCAACTACTCGACGTTCGAG (SEQ ID NO:31) | | |
| 29 | CAACTACTCGACGTTCGAGGTG (SEQ ID NO:32) | | |
| 24 | ACTCGACGTTCGAGGTGGGT (SEQ ID NO:2) | | |
| 175 | GCCAGTGAATTATTAATACGACTCAC TATAGGGAGAAGA (SEQ ID NO:33) | | |
| 564 | TCGCGCGTTTCGGTGATGAC (SEQ ID NO:34) | | |

Thus, the FRET primer assay disclosed herein has advantages over the SYBR Green^{®} assay, including but not limited to:
1) Less non-specific amplification signal - FRET primers require specific duplex dsDNA formation for fluorescence increase as opposed to non-specific dsDNA binding by SYBR Green^{®} dye;
2) Multiplex PCR capability - FRET primer can be labeled with different reporter dyes to allow multiplex PCR;
3) Better amplicon size and sequence differentiation - FRET primer assays produce labeled duplex double-stranded amplicons which may be identified by dissociation curve analysis. Better amplicon size and sequence differentiation was possible with the FRET primer assay.

### Example 7: The FRET primer assay can be performed using short annealing and extension time.

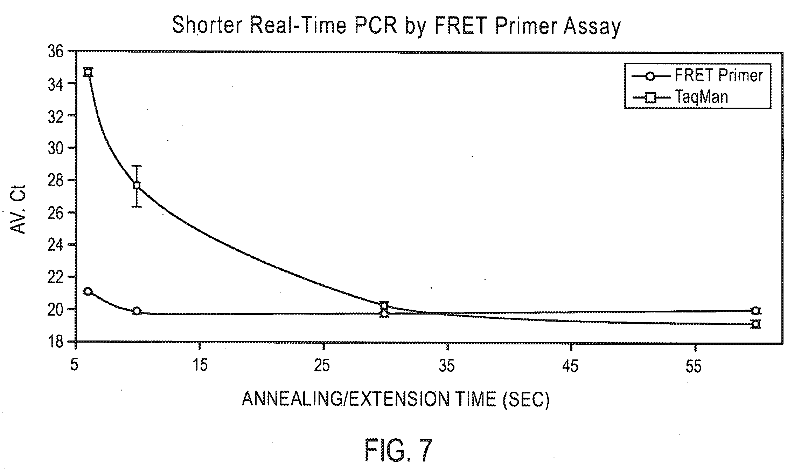

The TaqMan^{®} assay was compared to the FRET primer assay and analyzed by melting curve analysis using the probes and primers in Table 3. Figure 7 shows that the FRET primer assay enabled shorter annealing and extension times. The immediate fluorescence increase due to the ensuing rigid structure after extension of the FRET primer enabled faster PCR. The annealing/extension step for the same amplicon required 10 seconds for the FRET primer assay and 30 seconds for the TaqMan^{®} assay.

**Table 3: probes and primers used in FRET and TaqMan^{®} assays**

| | | |
|---|---|---|
| TaqMan^{®} | Forward | ATCTGGCCGGGCTTCAAC (SEQ ID NO:7) |
| | Probe | FAM-ATCCGTCGACTACTCG-MGB (SEQ ID NO:35) |
| | Reverse | GGTCACCCACCTCGAACGT (SEQ ID NO:4) |
| FRET Primer | Forward | ATCTGGCCGGGCTTCAAC (SEQ ID NO:7) |
| | Reverse | BHQ1-GGTCACCCACC(int-FAM T)CGAACGT (SEQ ID NO:6) |

Thus, the results in Examples 1, 2, and 7 show that the FRET primer assay provided herein has many advantages over the TaqMan^{®} assay, including but not limited to:
1) Primer design flexibility - the TaqMan^{®} assay requires three target sequences, forward primer probe and reverse primers. The FRET assays require only two target sequences, the forward and reverse primers. The makes the assay requirements less stringent and more flexible. This advantage is ideal for pathogen nucleic acid and small RNA sequences with limited target sites (i.e. Viral RNA sequences). Viral RNA sequences, due to their high mutation rate, contain very limited conserved target sequences;
2) Faster PCR - the FRET primer assay enables shorter annealing and extension times and the immediate fluorescence increase due to the ensuing rigid structure after extension of the FRET primer enables faster PCR (i.e. annealing extension steps for the same amplicon required 10 s. for FRET assay and 30 s. for TaqMan^{®});
3) Better Target sensitivity - The FRET primer assay enables higher fluorescence increases due to direct primer extension dependence as opposed to the TaqMan^{®} probe finding dependence. Higher fluorescence results in better detection sensitivity;
4) Target amplification verification - the FRET primer assays produce labeled duplex double-stranded amplicons which could be analyzed by dissociation curve analysis. Confirmation of target amplification as evidence by the dissociation curve analysis of the amplification peaks was able to reduce false negative amplification. TaqMan^{®} assays could not be analyzed by dissociation curve analysis and result in occasional false negatives due to the probe not binding;
5) Better SNP/subtype/mutation detection - the FRET primer assays produce labeled duplex double stranded amplicons which may be analyzed by dissociation curve analysis and high resolution melting curve analysis commonly used in SNP, subtype and mutation detection. Initial results indicate that better differentiation in amplicon size and sequence differentiation is possible with the FRET primer assays. In addition, a FRET primer with its 3' terminus targeting the SNP site may provide better SNPtyping differentiation since 3' mispriming is less tolerated by Taq polymerase;
6) Reduction in cost - A universal FRET-labeled primer tag may be functional for amplification of multiple target sequences. A FRET labeled primer tag can be appended to the 5' terminus of one of the target-specific sequence primers. The FRET labeled primer tag is a stretch of primer that does not bind to the target sequence but later on can be used as a binding site for a universal primer.

### Example 8: The FRET primer assay is not dependent on the specific Tag enzyme.

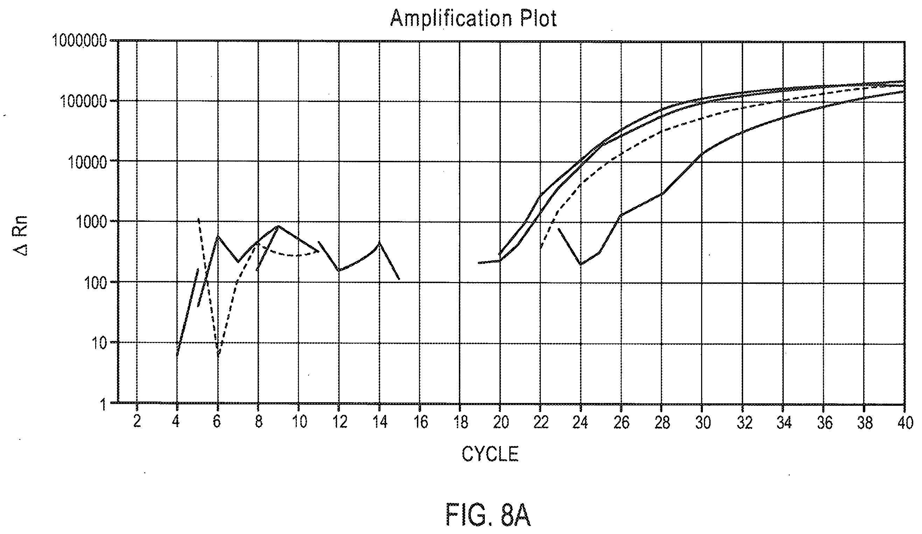

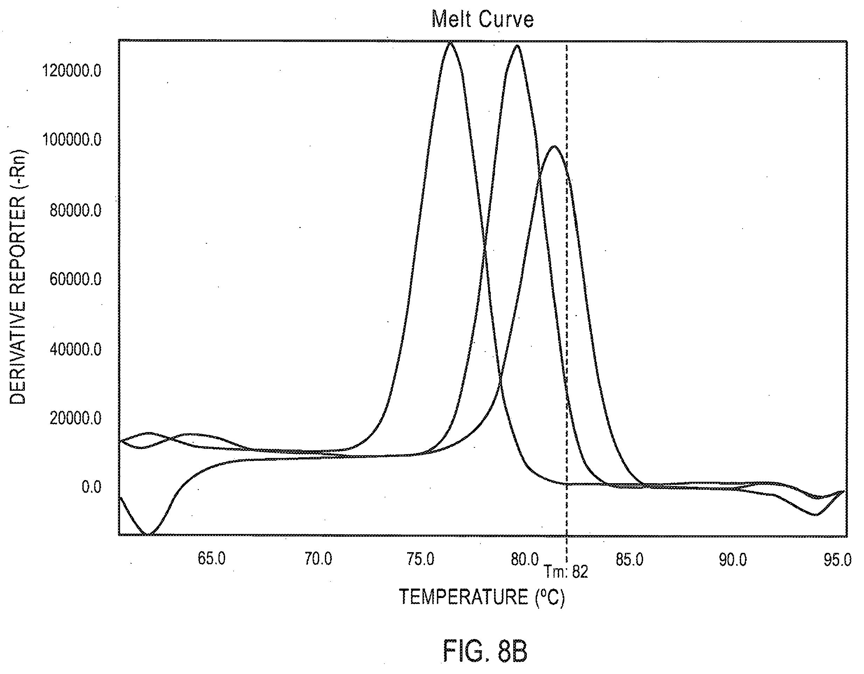

The FRET primer assay was tested with several different PCR reagent systems: Qiagen RT-PCR, Qiagen PCR, AB Uni PCR, and AgPath-ID™ PCR and all successfully quantified the EHV1 DNA target (see Figure 8A). As shown in Figure 10B, dissociation peaks differed due to the different buffer composition. All kits used the same pair of EHV1 FRET primers shown in Table 1.

### Example 9: The position of the fluorophore within the primer.

Assays were carried out to determine how the position of the FRET label within the primer affects the level of fluorescence. Typically, the highest fluorescence intensity is correlated with the best detection sensitivity. Thus, it was of interest to produce primers that resulted in the most sensitive assay. A number of FRET primers were produced and tested. These primers differed only in the position of the fluorophore. The primers are shown in Table 4. In the Table, the position of a FAM dye (a fluorescent dye) was varied depending on the position of a thymine in the primer. The forward primers that were tested included Durand 1-4. The reverse primers that were tested included Jang 1-4. The last column of Table 4 gives the number of nucleotides between the fluorophore (F*) and the quencher (Q). As shown in the Table, one FRET primer and one unlabeled primer was used for each reaction.

**Table 4: The position of the Thymine base labeled with a Fam dye.**

| **Target** | **Name** | **Forward 5'→3'** | **Reverse 5'→3'** | **# nt between F* & Q** |
|---|---|---|---|---|
| WSSV_ Durand_ 69 | Durand_1 | BHQ1-TGGTCCCGTCC(FAM- dT)CATCTCAG (SEQ ID NO:10) | GCTGCCTTGCCGGAAATTA (SEQ ID NO:15) | 11 |
| | Durand_2 | BHQ1- TGGTCCCGTCCTCATC(FAM- dT)CAG (SEQ ID NO:11) | | 16 |
| | Durand_3 | BHQ1-TGG(FAM- dT)CCCGTCCTCATCTCAG (SEQ ID NO:12) | | 3 |
| | Durand_4 | BHQ1-TGGTCCCGTCTCA(FAM- dT)CTCAG (SEQ ID NO:13) | | 13 |
| WSSV_ Jang_ 154 | Jang_1 | CCAGTTCAGAATCGGACGTT (SEQ ID NO:14) | BHQ1-AAAGACGCC(FAM- dT)ACCCTGTTGA (SEQ ID NO:16) | 9 |
| | Jang_2 | | BHQ1- AAAGACGCCTACCCTGT(FAM- dT)GA (SEQ ID NO:17) | 17 |
| | Jang_3 | | BHQ1-AAAGACGCCTACCC(FAM- dT)GTTGA (SEQ ID NO:18) | 14 |
| | Jang_4 | | BHQ1- AAAGACGCCTACCCTG(FAM- dT)TGA (SEQ ID NO:19) | 16 |

The assays were performed on an ABI Fast 7500™ sequence detection system with VetMAX™ qPCR master mix (Ambion). The cycling consisted of 10 min at 95°C, followed by 40 cycles of 95°C for 15s, and 60°C for 1 min using Standard 7500 run mode and instrument default dissociation melt protocol at 95°C for 15s, 60°C for 1min, 95°C for 15s, 60°C for 15s. A dissociation stage was added to observe for any specific or non specific amplification in the No Template Control (NTC). The FRET primers that gave the best linearity, limit of detection (LOD) and Cₜ were selected to evaluate the feasibility of running fast cycling.

The Whit Spot Syndrome Virus (WSSV) DNA template (WSSV DNA Sequence: Accession No U50923 Sequence Range: 781-1280) was cloned into a Pdp19 vector and synthesized from Blue Heron Technology, Inc. All labeled and unlabeled primers were obtained from Biosearch Technologies, Inc. Each labeled and unlabeled primer was used at a concentration of 0.5µM in a final reaction volume of 25 µL. The unlabeled primers and Taqman probe (Table 5) were obtained from Applied Biosystems and were included for general comparison. Each unlabeled primer was used at 0.5 µM and the Taqman probe was used at 0.25 µM in a final reaction volume of 25 µL.

### Example 10: Testing the primers in a TagMan^{®} assay.

Three different thermal protocols were run to determine how the FRET primers performed in fast cycling. The assays were performed on an ABI Fast 7500™ sequence detection system with VetMAX™ qPCR master mix (Ambion). The cycling protocol consisted of (1) 10 min at 95°C, followed by 40 cycles of 95°C for 15s, and 60°C for 1 min using Standard 7500 mode and the instrument default dissociation melt protocol at 95°C for 15s, 60°C for 1min, 95°C for 15s, 60°C for 15s; (2) 10min at 95°C, followed by 40 cycles of 95°C for 2s, and 60°C for 40s using Fast 7500 mode and instrument default dissociation melt protocol at 95°C for 15s, 60°C for 1min, 95°C for 15s, 60°C for 15s; and (3) 10min at 95°C, followed by 40 cycles of 95°C for 3s, and 60°C for 40s using Fast 7500 mode and instrument default dissociation melt protocol at 95°C for 15s, 60°C for 1min, 95°C for 15s, 60°C for 15s. The data acquisition and analysis were carried out with ABI Fast 7500™ sequence detector software (SDS 1.4). The Taq primers are shown below.

Using the several possibilities for attachment of the FRET dye (the thymine bases in the primers) primers were tested. The best position to label the FRET dye was determined using the primers in Table 5. The results showed that the optimum position to label the FRET dye to acquire the strongest signal and best Cₜ was at the thymine base that was furthest away from the quencher. In this study, for the Durand assay, the Durand_2 primer showed the best Ct and ΔRxn (Table 6A and 6B). Thus, the optimum position to label the Fam dye was 16 nucleotides from the quencher. For the Jang assay, the Jang_2 primer showed the best Ct and ΔRxn (Table 6A and 6B). Thus, the optimum position to label the Fam dye for the Jang assay, was 17 nucleotides from the quencher. In Jang assay, 17 nucleotides and 16 nucleotides from the quencher gave comparable Cₜ and fluorescence with 17 nucleotides giving a slightly higher fluorescence signal.

FRET primers enable shorter annealing and extension times due to the immediate fluorescence increase due to direct primer extension as compared to the TaqMan^{®} probe binding. To determine if FRET primers can run faster PCR, the same amplicon was amplified using FRET and TaqMan^{®} assay in both fast and standard PCR conditions. Under standard PCR run conditions, both FRET and TaqMan^{®} assay gave comparable PCR efficiency and correlation coefficients. However, under fast PCR run condition with annealing step at 30s at instrument fast ramp rate, the FRET assay gave a PCR efficiency of 91% as compared to the TaqMan^{®} assay that gave a PCR efficiency of 83%.

**Table 5: Taq primers used in the TaqMan^{®} assay**

| **Target** | **Forward 5'→3'** | **Reverse 5'→3'** | **Probe 5'→3'** |
|---|---|---|---|
| WSSV_ Durand_ 69 | TGGTCCCGTCCTCATCT CAG (SEQ ID NO:20) | GCTGCCTTGCCGGAAA TTA (SEQ ID NO:22) | Fam- AGCCATGAAGAATGCCGTCTATC ACACA- NFQ (SEQ ID NO:24) |
| WSSV_ Jang_154 | CCAGTTCAGAATCGGA CGTT (SEQ ID NO:21) | AAAGACGCCTACCCTG TTGA (SEQ ID NO:23) | Fam- TCCATAGTTCCTGGTTTGTAATGT GCCG – NFQ (SEQ ID NO:25) |

Tables 6A-6B show the fluorescence signals obtained for the Durand Forward FRET primers, the Jang Reverse FRET primers, the Durand Forward FRET primers, and the Jang Reverse FRET primers as compared to a TaqMan^{®} assay at a variety of concentrations. Seven different copy numbers were tested and the value dRN and standard deviation are shown in Tables 6A and 6B for those 7 copy numbers.

**Table 6A: Cₜ of serially diluted WSSV amplicons using primers listed in Table 5:**

| **Primer** | **Copy Number** | **Ct** |
|---|---|---|
| | 6.25 | 35.27 |
| | 12.5 | 33.85 |
| | 25 | 32.62 |
| Durand_1 | 50 | 31.92 |
| | 100 | 30.82 |
| | 1000 | 27.39 |
| | 10000 | 24.01 |
| | 6.25 | 33.19 |
| | 12.5 | 33.05 |
| | 25 | 31.68 |
| Durand_2 | 50 | 30.37 |
| | 100 | 29.20 |
| | 1000 | 25.86 |
| | 10000 | 22.42 |
| | 6.25 | 40.00 |
| | 12.5 | 40.00 |
| | 25 | 40.00 |
| Durand_3 | 50 | 40.00 |
| | 100 | 40.00 |
| | 1000 | 40.00 |
| | 10000 | 40.00 |
| | 6.25 | 34.31 |
| | 12.5 | 34.06 |
| | 25 | 32.90 |
| Durand_4 | 50 | 31.55 |
| | 100 | 30.65 |
| | 1000 | 27.22 |
| | 10000 | 23.81 |
| | 6.25 | 35.66 |
| | 12.5 | 33.05 |
| | 25 | 32.07 |
| Durand_Taqman | 50 | 30.99 |
| | 100 | 29.55 |
| | 1000 | 26.30 |
| | 10000 | 22.89 |
| | 6.25 | 37.34 |
| | 12.5 | 35.71 |
| | 25 | 34.57 |
| Jang 1 | 50 | 33.15 |
| | 100 | 32.57 |
| | 1000 | 29.31 |
| | 10000 | 25.64 |
| | 6.25 | 34.75 |
| | 12.5 | 32.79 |
| Jang 2 | 25 | 31.87 |
| | 50 | 31.09 |
| | 100 | 30.04 |
| | 1000 | 27.04 |
| | 10000 | 23.33 |
| | 6.25 | 36.14 |
| | 12.5 | 35.12 |
| | 25 | 33.07 |
| Jang 3 | 50 | 32.32 |
| | 100 | 30.98 |
| | 1000 | 27.94 |
| | 10000 | 24.33 |
| | 6.25 | 34.82 |
| | 12.5 | 33.85 |
| | 25 | 33.56 |
| Jang 4 | 50 | 31.26 |
| | 100 | 29.91 |
| | 1000 | 27.11 |
| | 10000 | 23.75 |
| | 6.25 | 34.94 |
| | 12.5 | 34.53 |
| | 25 | 31.24 |
| Jang Taqman | 50 | 31.19 |
| | 100 | 30.19 |
| | 1000 | 26.89 |
| | 10000 | 23.41 |

**Table 6B: ΔRxn of serially diluted WSSV amplicons using primers listed in Table 5**

| **Reporter Dye** | **Copy Number** | **ΔRn** |
|---|---|---|
| | 6.25 | 0.66 |
| | 12.5 | 0.87 |
| | 25 | 0.96 |
| Durand 1 | 50 | 1.31 |
| | 100 | 1.38 |
| | 1000 | 2.18 |
| | 10000 | 3.08 |
| | 6.25 | 1.26 |
| | 12.5 | 1.96 |
| | 25 | 2.24 |
| Durand 2 | 50 | 2.67 |
| | 100 | 3.07 |
| | 1000 | 4.78 |
| | 10000 | 6.55 |
| | 6.25 | 0.10 |
| | 12.5 | 0.17 |
| | 25 | 0.24 |
| Durand 3 | 50 | 0.28 |
| | 100 | 0.44 |
| | 1000 | 0.61 |
| | 10000 | 0.83 |
| | 6.25 | 0.04 |
| | 12.5 | 0.04 |
| | 25 | 0.08 |
| Durand 4 | 50 | 0.12 |
| | 100 | 0.12 |
| | 1000 | 0.18 |
| | 10000 | 0.25 |
| Durand Taqman | 6.25 | 1.13 |
| | 12.5 | 1.41 |
| | 25 | 1.88 |
| | 50 | 2.09 |
| | 100 | 2.67 |
| | 1000 | 3.97 |
| | 10000 | 5.03 |
| | 6.25 | 0.39 |
| | 12.5 | 0.70 |
| | 25 | 0.91 |
| Jang 1 | 50 | 1.23 |
| | 100 | 1.36 |
| | 1000 | 2.04 |
| | 10000 | 2.84 |
| | 6.25 | 2.01 |
| | 12.5 | 3.30 |
| | 25 | 4.12 |
| Jang 2 | 50 | 4.70 |
| | 100 | 5.73 |
| | 1000 | 8.28 |
| | 10000 | 11.81 |
| | 6.25 | 0.86 |
| | 12.5 | 1.15 |
| | 25 | 2.02 |
| Jang 3 | 50 | 2.31 |
| | 100 | 2.92 |
| | 1000 | 4.28 |
| | 10000 | 5.81 |
| | 6.25 | 1.76 |
| | 12.5 | 2.26 |
| | 25 | 2.57 |
| Jang 4 | 50 | 4.27 |
| | 100 | 5.17 |
| | 1000 | 7.55 |
| | 10000 | 10.82 |
| Jang Taqman | 6.25 | 2.57 |
| | 12.5 | 2.83 |
| | 25 | 5.28 |
| | 50 | 6.29 |
| | 100 | 8.05 |
| | 1000 | 12.39 |
| | 10000 | 16.90 |

Each embodiment disclosed herein may be used or otherwise combined with any of the other embodiments disclosed. Any element of any embodiment may be used in any embodiment.

Although the invention has been described with reference to specific embodiments, it will be understood by those skilled in the art that various changes may be made and equivalents may be substituted for elements thereof without departing from the scope of the invention. In addition, modifications may be made without departing from the essential teachings of the invention.

## Claims

1. A method for the quantification or detection of one or more target nucleic acid molecules in a sample during nucleic acid synthesis, the method comprising:
a) mixing one or more target nucleic acid molecules with one or more single stranded oligonucleotide primers complementary to a portion of said one or more target nucleic acid molecules
wherein each primer is a fluorescently-labeled oligonucleotide, labeled with a fluorophore and a quencher; wherein one or both of the fluorophore and quencher are internally located in the primer and wherein the Quencher is located near or at the 5'-end;
b) incubating said mixture with a polymerase under conditions sufficient to synthesize one or more nucleic acid molecules complementary to all or a portion of said one or more target nucleic acid molecules, said one or more synthesized nucleic acid molecules comprising said one or more oligonucleotides , wherein the extension of said one or more primers by said polymerase by at least 3 nucleotides releases fluorescence; and
c) detecting the presence or absence or quantifying the amount of said one or more synthesized nucleic acid molecules by measuring said-fluorescense.

2. A method for amplifying a double-stranded nucleic acid molecule, comprising:
a) providing a first and second primer, wherein said first primer is complementary to a sequence within or at or near the 3'-terminus of the first strand of said nucleic acid molecule and said second primer is complementary to a sequence within or at or near the 3'-terminus of the second strand of said nucleic acid molecule;
b) hybridizing said first primer to said first strand and said second primer to said second strand in the presence of one or more polymerases, under conditions such that said primers are extended to result in the synthesis of a third nucleic acid molecule complementary to all or a portion of said first strand and a fourth nucleic acid molecule complementary to all or a portion of said second strand;
c) denaturing said first and third strands, and said second and fourth strands; and repeating the above steps one or more times, wherein one of said first and second primers is dual-labeled with a fluorophore and a quencher; wherein one or both of the fluorophore and quencher are internally located in the primer and wherein the Quencher is located near or at the 5'-end; and wherein said dual-labeled primer undergoes a detectable change in fluorescence upon extension of said one or more labeled primers to said nucleic acid molecule, wherein extension is by at least 3 nucleotides.

3. The method of claim 1 or 2, wherein steps (a), (b), and (c) are performed simultaneously or separately in any order.

4. The method of claim 1 or 2, wherein step (c) is performed in the presence of unincorporated fluorescently-labeled oligonucleotides.

5. The method of claim 1 or 2, wherein no additional treatment steps are necessary between steps (b) and (c) or concomitant with step (c), optionally wherein the additional treatment steps are selected from the group consisting of gel electrophoresis, immobilization of amplification product and washing away of unincorporated oligonucleotide, digestion or cleavage of the oligonucleotide, 3'→5' exonuclease treatment, denaturation and heat treatment.

6. The method of claim 1 or 2, wherein the quencher and fluorophore are separated at a distance such that when the oligonucleotide is not bound to the target nucleic acid the fluorophore is quenched by the quencher and when the oligonucleotide is bound to the target nucleic acid and extended by at least three nucleotides the fluorophore is not quenched by the quencher.

7. The method of claim 1, wherein the distance is between about 3 and 20 nucleotides or the method of claim 2 wherein the distance is between about 4 and 20 nucleotides.

8. The method of claim 1, wherein the distance is between about 6 and 19 nucleotides.

9. The method of claim 1 or 2, wherein the fluorophore is selected from the group consisting of fluorescein, 5-carboxyfluorescein (FAM), 2'7'-dimethoxy-4'5'-dichloro-6- carboxyfluorescein (JOE), rhodamine, 6-carboxyrhodamine (R6G),N,N,N',N'-tetramethyl-6- carboxyrhodamine (TAMRA), 6-carboxy-X-rhodamine (ROX), 4-(4'-dimethylaminophenylazo) benzoic acid (DABCYL), and 5-(2'-aminoethyl)aminonaphthalene-1-sulfonic acid (EDANS) and/or wherein the quencher is selected from the group consisting of a Black Hole Quencher^{®}, an Iowa Black^{®} quencher, an Eclipse^{®} Dark quencher and a DABCYL quencher and a derivative thereof.

10. The method of claim 1, wherein the fluorophore is internally located on said oligonucleotide and the quencher is located on the 5' end of said oligonucleotide, or the method of claim 2, wherein the fluorophore is located on an internal nucleotide and the quencher is on the 5' end of the dual-labeled primer.

11. The method of claim 1 or 2, wherein said target nucleic acid is about 15 to 100 nucleotides in length.

12. The method of claim 1 or 2, wherein the detection is performed using a real-time PCR instrument, preferably a spectrophotometric real-time PCR instrument.

13. The method of claim 1 or 2, wherein the target nucleic acid is selected from the group consisting of genomic DNA, RNA, cDNA, mRNA, and chemically synthesized DNA, optionally wherein the target nucleic acid is a sequence of an infectious disease agent, wild-type human genomic sequence, or a mutation implicated in a human disease or disorder.

14. The method of claim 1, further comprising denaturing the product of step (b) and incubating under conditions sufficient to synthesize one or more nucleic acid molecules complementary to all or a portion of said one or more target nucleic acid molecules, said one or more synthesized nucleic acid molecules comprising said one or more oligonucleotides, and optionally repeating the denaturing and incubating one or more times.

15. A method for the quantification or detection of one or more target nucleic acid molecules in a sample during nucleic acid synthesis, the method comprising:
a)mixing one or more target nucleic acid molecules with one or more single stranded oligonucleotide primers complementary to a portion of said one or more target nucleic acid molecules
wherein each primer is a fluorescently-labeled oligonucleotide, labeled with a fluorophore and a quencher; wherein one or both of the fluorophore and quencher are located in part of the primer that is complementary to the target; wherein one or both of the fluorophore and quencher are internally located in the primer and wherein the Quencher is located near or at the 5'-end;
b)incubating said mixture with a polymerase under conditions sufficient to synthesize one or more nucleic acid molecules complementary to all or a portion of said one or more target nucleic acid molecules, said one or more synthesized nucleic acid molecules comprising said one or more oligonucleotides , wherein the extension of said one or more primers by said polymerase by at least 3 nucleotides releases fluorescence; and
c)detecting the presence or absence or quantifying the amount of said one or more synthesized nucleic acid molecules by measuring said fluorescense.

16. A fluorescently-labeled oligonucleotide comprising both a fluorophore and a quencher, wherein the fluorophore is attached to the oligonucleotide internally, near the 5' end or at the 5' end, and the quencher is attached to the oligonucleotide internally, near the 5'end, or at the 5' end, and wherein the fluorophore and the quencher are separated at a distance such that when the oligonucleotide is bound to a target nucleic acid and extended by at least three nucleotides the fluorophore is not quenched by the quencher, and when the oligonucleotide is not bound to a target nucleic acid the fluorophore is quenched by the quencher, wherein the distance is between 3 and 20 nucleotides, or between 6 and 19 nucleotides.

17. The oligonucleotide of claim 16, wherein the fluorophore is selected from the group consisting of fluorescein, 5-carboxyfluorescein (FAM), 2'7'-dimethoxy-4'5'-dichloro-6- carboxyfluorescein (JOE), rhodamine, 6-carboxyrhodamine (R6G), N,N,N',N'-tetramethyl-6- carboxyrhodamine (TAMRA), 6-carboxy-X-rhodamine (ROX), 4-(4'-dimethylaminophenylazo) benzoic acid (DABCYL), and 5-(2'-aminoethyl)aminonaphthalene-1-sulfonic acid (EDANS) and/or wherein the quencher is selected from the group consisting of a Black Hole Quencher^{®},an Iowa Black^{®} quencher, an Eclipse^{®} Dark quencher and a DABCYL quencher and a derivative thereof.

18. A kit for the quantification or detection of one or more target nucleic acid molecules in a sample during nucleic acid synthesis, comprising:
(a) a polymerase, and
(b) a dual-labeled oligonucleotide comprising a fluorophore and a quencher,
wherein one or both of the fluorophore and quencher are internally located in the primer and wherein the Quencher is located near or at the 5'-end; wherein the quencher and fluorophore are separated at a distance such that when the dual-labeled oligonucleotide bound to the nucleic acid molecule is not extended the fluorophore is quenched by the quencher and when the dual-labeled oligonucleotide bound to the nucleic acid molecule is extended the fluorophore is not quenched by the quencher and wherein the distance is between 3 and 20 nucleotides.

19. A composition comprising:
(a) a polymerase, and
(b) a dual-labeled oligonucleotide comprising a fluorophore and a quencher,
wherein one or both of the fluorophore and quencher are internally located in the primer and wherein the Quencher is located near or at the 5'-end; wherein the quencher and fluorophore are separated at a distance such that when the dual-labeled oligonucleotide bound to the nucleic acid molecule is not extended the fluorophore is quenched by the quencher and when the dual-labeled oligonucleotide bound to the nucleic acid molecule is extended the fluorophore is not quenched by the quencher and wherein the distance is between 3 and 20 nucleotides.

## Patentansprüche

1. Verfahren zum Quantifizieren oder Nachweisen eines oder mehrerer Zielnukleinsäuremoleküle in einer Probe während einer Nukleinsäuresynthese, wobei das Verfahren umfasst:
a) Mischen eines oder mehrerer Zielnukleinsäuremoleküle mit einem oder mehreren einzelsträngigen Oligonukleotidprimern, die zu einem Abschnitt des einen oder der mehreren Zielnukleinsäuremoleküle komplementär sind,
wobei jeder Primer ein fluoreszierend markiertes Oligonukleotid ist, das mit einem Fluorophor und einem Quencher markiert ist; wobei eines oder beides des Fluorophors und Quenchers im Inneren des Primers angeordnet sind, und wobei der Quencher nahe dem 5'-Ende oder an diesem angeordnet ist;
b) Inkubieren der Mischung mit einer Polymerase unter Bedingungen, die ausreichend sind, um ein oder mehrere Nukleinsäuremoleküle zu synthetisieren, die zu dem einen oder den mehreren Zielnukleinsäuremolekülen als Ganzes oder einem Abschnitt davon komplementär sind, wobei das eine oder die mehreren synthetisierten Nukleinsäuremoleküle das eine oder die mehreren Oligonukleotide umfassen, wobei die Verlängerung des einen oder der mehreren Primer durch die Polymerase um mindestens 3 Nukleotide Fluoreszenz freisetzt; und
c) Nachweisen des Vorhandenseins oder Nichtvorhandenseins oder Quantifizieren der Menge des einen oder der mehreren synthetisierten Nukleinsäuremoleküle durch Messen der Fluoreszenz.

2. Verfahren zum Amplifizieren eines doppelsträngigen Nukleinsäuremoleküls, das umfasst:
a) Bereitstellen eines ersten und eines zweiten Primers, wobei der erste Primer zu einer Sequenz innerhalb des 3'-Terminus des ersten Strangs des Nukleinsäuremoleküls oder an diesem oder nahe diesem komplementär ist und der zweite Primer zu einer Sequenz innerhalb des 3'-Terminus des zweiten Strangs des Nukleinsäuremoleküls oder an diesem oder nahe diesem komplementär ist;
b) Hybridisieren des ersten Primers mit dem ersten Strang und des zweiten Primers mit dem zweiten Strang in der Gegenwart einer oder mehrerer Polymerasen unter Bedingungen, so dass die Primer verlängert werden, um die Synthese eines dritten Nukleinsäuremoleküls, das zum ersten Strang als Ganzes oder einem Abschnitt davon komplementär ist, und eines vierten Nukleinsäuremoleküls, das zum zweiten Strang als Ganzes oder einem Abschnitt davon komplementär ist, zu erzielen;
c) Denaturieren der ersten und dritten Stränge und der zweiten und vierten Stränge; und Wiederholen der obigen Schritte einmal oder mehrmals, wobei einer des ersten und des zweiten Primers mit einem Fluorophor und einem Quencher doppelt markiert ist; wobei eines oder beide des Fluorophors und Quenchers innerhalb des Primers angeordnet sind und wobei der Quencher nahe dem 5'-Ende oder an diesem angeordnet ist; und wobei der doppelt markierte Primer eine nachweisbare Veränderung der Fluoreszenz bei Verlängerung des einen oder der mehreren markierten Primer zum Nukleinsäuremolekül erfährt, wobei die Verlängerung um mindestens 3 Nukleotide erfolgt.

3. Verfahren nach Anspruch 1 oder 2, wobei die Schritte (a), (b) und (c) gleichzeitig oder getrennt in einer beliebigen Reihenfolge durchgeführt werden.

4. Verfahren nach Anspruch 1 oder 2, wobei Schritt (c) in der Gegenwart von nichteingebauten fluoreszierend markierten Oligonukleotiden durchgeführt wird.

5. Verfahren nach Anspruch 1 oder 2, wobei keine weiteren Behandlungsschritte zwischen den Schritten (b) und (c) oder gleichzeitig mit Schritt (c) erforderlich sind, optional wobei die weiteren Behandlungsschritte aus der Gruppe, bestehend aus Gelelektrophorese, Immobilisierung eines Amplifikationsprodukts und Wegwaschen von nicht-eingebautem Oligonukleotid, Verdau oder Spaltung des Oligonukleotids, Behandlung mit 3'- -> 5'-Exonuklease, Denaturierung und Wärmebehandlung ausgewählt sind.

6. Verfahren nach Anspruch 1 oder 2, wobei der Quencher und das Fluorophor mit einem Abstand getrennt sind, so dass, wenn das Oligonukleotid nicht an die Zielnukleinsäure gebunden ist, das Fluorophor vom Quencher gequencht wird, und wenn das Oligonukleotid an die Zielnukleinsäure gebunden und um mindestens 3 Nukleotide verlängert ist, das Fluorophor vom Quencher nicht gequencht wird.

7. Verfahren nach Anspruch 1, wobei der Abstand zwischen ungefähr 3 und 20 Nukleotiden beträgt, oder Verfahren nach Anspruch 2, wobei der Abstand zwischen ungefähr 4 und 20 Nukleotiden beträgt.

8. Verfahren nach Anspruch 1, wobei der Abstand zwischen ungefähr 6 und 19 Nukleotiden beträgt.

9. Verfahren nach Anspruch 1 oder 2, wobei das Fluorophor aus der Gruppe, bestehend aus Fluorescein, 5-Carboxyfluorescein (FAM), 2'7'-Dimethoxy-4'5'-dichlor-6-carboxyfluorescein (JOE), Rhodamin, 6-Carboxyrhodamin (R6G), N,N,N',N'-Tetramethyl-6-carboxyrhodamin (TAMRA), 6-Carboxy-X-rhodamin (ROX), 4-(4'-Dimethylaminophenylazo)benzoesäure (DABCYL) und 5-(2'-Aminoethyl)aminonaphthalen-1-sulfonsäure (EDANS), ausgewählt ist und/oder wobei der Quencher aus der Gruppe, bestehend aus einem Black Hole Quencher®, einem Iowa Black®-Quencher, einem Eclipse® Dark-Quencher und einem DABCYL-Quencher und einem Derivat davon ausgewählt ist.

10. Verfahren nach Anspruch 1, wobei das Fluorophor im Inneren am Oligonukleotid angeordnet ist und der Quencher am 5'-Ende des Oligonukleotids angeordnet ist, oder Verfahren nach Anspruch 2, wobei das Fluorophor an einem inneren Nukleotid angeordnet ist und der Quencher am 5'-Ende des doppelt markierten Primers angeordnet ist.

11. Verfahren nach Anspruch 1 oder 2, wobei die Zielnukleinsäure ungefähr 15 bis 100 Nukleotide lang ist.

12. Verfahren nach Anspruch 1 oder 2, wobei der Nachweis unter Verwendung eines Echtzeit-PCR-Instruments durchgeführt wird, vorzugsweise eines spektrophotometrischen Echtzeit-PCR-Instruments.

13. Verfahren nach Anspruch 1 oder 2, wobei die Zielnukleinsäure aus der Gruppe, bestehend aus genomischer DNA, RNA, cDNA, mRNA und chemisch synthetisierter DNA, optional wobei die Zielnukleinsäure eine Sequenz eines Infektionskrankheitserregers, eine humane genomische Wildtyp-Sequenz oder Mutation, die mit einer humanen Krankheit oder Störung in Verbindung gebracht wird, ausgewählt ist.

14. Verfahren nach Anspruch 1, das des Weiteren das Denaturieren des Produkts von Schritt (b) und das Inkubieren unter Bedingungen, die ausreichend sind, um ein oder mehrere Nukleinsäuremoleküle zu synthetisieren, die zu der einen oder den mehreren Zielnukleinsäuremolekülen als Ganzes oder einem Abschnitt davon komplementär sind, wobei das eine oder die mehreren synthetisierten Nukleinsäuremoleküle das eine oder die mehreren Oligonukleotide umfassen, und optional das Wiederholen des Denaturierens und Inkubierens einmal oder mehrmals umfasst.

15. Verfahren zum Quantifizieren oder Nachweisen eines oder mehrerer Zielnukleinsäuremoleküle in einer Probe während einer Nukleinsäuresynthese, wobei das Verfahren umfasst:
a) Mischen eines oder mehrerer Zielnukleinsäuremoleküle mit einem oder mehreren einzelsträngigen Oligonukleotidprimern, die zu einem Abschnitt des einen oder der mehreren Zielnukleinsäuremoleküle komplementär sind,
wobei jeder Primer ein fluoreszierend markiertes Oligonukleotid ist, das mit einem Fluorophor und einem Quencher markiert ist; wobei eines oder beides des Fluorophors und Quenchers in einem Teil des Primers angeordnet sind, der zum Ziel komplementär ist; wobei eines oder beide des Fluorophors und Quenchers im Inneren des Primers angeordnet sind, und wobei der Quencher nahe dem 5'-Ende oder an diesem angeordnet ist;
b) Inkubieren der Mischung mit einer Polymerase unter Bedingungen, die ausreichend sind, um ein oder mehrere Nukleinsäuremoleküle zu synthetisieren, die zu dem einen oder den mehreren Zielnukleinsäuremolekülen als Ganzes oder einem Abschnitt davon komplementär sind, wobei das eine oder die mehreren synthetisierten Nukleinsäuremoleküle das eine oder die mehreren Oligonukleotide umfassen, wobei die Verlängerung des einen oder der mehreren Primer durch die Polymerase um mindestens 3 Nukleotide Fluoreszenz freisetzt; und
c) Nachweisen des Vorhandenseins oder Nichtvorhandenseins oder Quantifizieren der Menge des einen oder der mehreren synthetisierten Nukleinsäuremoleküle durch Messen der Fluoreszenz.

16. Fluoreszierend markiertes Oligonukleotid, das sowohl ein Fluorophor als auch einen Quencher umfasst, wobei das Fluorophor innen nahe dem 5'-Ende oder am 5'-Ende an das Oligonukleotid gebunden ist und wobei der Quencher innen nahe dem 5'-Ende oder am 5'-Ende am Oligonukleotid gebunden ist, und wobei das Fluorophor und der Quencher mit einem Abstand getrennt sind, so dass, wenn das Oligonukleotid an eine Zielnukleinsäure gebunden und um mindestens 3 Nukleotide verlängert ist, das Fluorophor vom Quencher nicht gequencht wird, und wenn das Oligonukleotid nicht an eine Zielnukleinsäure gebunden ist, das Fluorophor vom Quencher gequencht wird, wobei der Abstand zwischen 3 und 20 Nukleotiden oder zwischen 6 und 19 Nukleotiden beträgt.

17. Oligonukleotid nach Anspruch 16, wobei das Fluorophor aus der Gruppe, bestehend aus Fluorescein, 5-Carboxyfluorescein (FAM), 2'7'-Dimethoxy-4'5'-dichlor-6-carboxyfluorescein (JOE), Rhodamin, 6-Carboxyrhodamin (R6G), N,N,N',N'-Tetramethyl-6-carboxyrhodamin (TAMRA), 6-Carboxy-X-rhodamin (ROX), 4-(4'-Dimethylaminophenylazo)benzoesäure (DABCYL) und 5-(2'-Aminoethyl)aminonaphthalen-1-sulfonsäure (EDANS) ausgewählt ist, und/oder wobei der Quencher aus der Gruppe, bestehend aus einem Black Hole Quencher®, einem Iowa Black®-Quencher, einem Eclipse® Dark-Quencher und einem DABCYL-Quencher und einem Derivat davon ausgewählt ist.

18. Kit zum Quantifizieren oder Nachweisen eines oder mehrerer Zielnukleinsäuremoleküle in einer Probe während einer Nukleinsäuresynthese, der umfasst:
(a) eine Polymerase, und
(b) ein doppelt markiertes Oligonukleotid, das ein Fluorophor und einen Quencher umfasst, wobei eines oder beide des Fluorophors und des Quenchers im Inneren des Primers angeordnet sind, und wobei der Quencher nahe dem 5'-Ende oder an diesem angeordnet ist; wobei der Quencher und das Fluorophor mit einem Abstand getrennt sind, so dass, wenn das doppelt markierte Oligonukleotid, das an das Zielnukleinsäuremolekül gebunden ist, nicht verlängert ist, das Fluorophor vom Quencher gequencht wird, und wenn das doppelt markierte Oligonukleotid, das an das Nukleinsäuremolekül gebunden ist, verlängert ist, das Fluorophor nicht vom Quencher gequencht wird, und wobei der Abstand zwischen 3 und 20 Nukleotiden beträgt.

19. Zusammensetzung, die umfasst:
(a) eine Polymerase, und
(b) ein doppelt markiertes Oligonukleotid, das ein Fluorophor und einen Quencher umfasst, wobei eines oder beide des Fluorophors und des Quenchers im Inneren des Primers angeordnet sind, und wobei der Quencher nahe dem 5'-Ende oder an diesem angeordnet ist; wobei der Quencher und das Fluorophor mit einem Abstand getrennt sind, so dass, wenn das doppelt markierte Oligonukleotid, das an das Zielnukleinsäuremolekül gebunden ist, nicht verlängert ist, das Fluorophor vom Quencher gequencht wird, und wenn das doppelt markierte Oligonukleotid, das an das Nukleinsäuremolekül gebunden ist, verlängert ist, das Fluorophor nicht vom Quencher gequencht wird, und wobei der Abstand zwischen 3 und 20 Nukleotiden beträgt.

## Revendications

1. Procédé de quantification ou de détection d'une ou de plusieurs molécules d'acide nucléique cibles dans un échantillon lors de la synthèse d'acide nucléique, le procédé comprenant les opérations consistant à :
a) mélanger une ou plusieurs molécules d'acide nucléique cibles à une ou plusieurs amorces oligonucléotidiques simples brins complémentaires d'une partie desdites une ou plusieurs molécules d'acide nucléique cibles,
chaque amorce étant un oligonucléotide marqué par fluorescence, marqué avec un fluorophore et un désactivateur ; l'un des fluorophore et désactivateur ou les deux étant situés à l'intérieur de l'amorce, et le désactivateur étant situé à l'extrémité 5' ou à proximité de celle-ci;
b) incuber ledit mélange avec une polymérase dans des conditions suffisantes pour synthétiser une ou plusieurs molécules d'acide nucléique complémentaires de la totalité ou d'une partie desdites une ou plusieurs molécules d'acide nucléique cibles, lesdites une ou plusieurs molécules d'acide nucléique synthétisées comprenant lesdits un ou plusieurs oligonucléotides, l'extension d'au moins 3 nucléotides desdites une ou plusieurs amorces par ladite polymérase déclenchant de la fluorescence ; et
c) détecter la présence ou l'absence, ou quantifier la quantité, desdites une ou plusieurs molécules d'acide nucléique synthétisées par mesure de ladite fluorescence.

2. Procédé d'amplification d'une molécule d'acide nucléique double brin, comprenant les opérations consistant à :
a) produire une première et une seconde amorce, ladite première amorce étant complémentaire d'une séquence à l'extrémité 3' du premier brin de ladite molécule d'acide nucléique ou à l'intérieur ou à proximité de celle-ci et ladite seconde amorce étant complémentaire d'une séquence à l'extrémité 3' du deuxième brin de ladite molécule d'acide nucléique ou à l'intérieur ou proximité de celle-ci ;
b) hybrider ladite première amorce audit premier brin et ladite seconde amorce audit deuxième brin en présence d'une ou de plusieurs polymérases dans des conditions telles que lesdites amorces sont étendues pour aboutir à la synthèse d'une troisième molécule d'acide nucléique complémentaire de la totalité ou d'une partie dudit premier brin et d'une quatrième molécule d'acide nucléique complémentaire de la totalité ou d'une partie dudit second brin ;
c) dénaturer lesdits premier et troisième brins et lesdits deuxième et quatrième brins ; et répéter les étapes ci-dessus une ou plusieurs fois, une desdites première et seconde amorces étant doublement marquées avec un fluorophore et un désactivateur ; le fluorophore ou le désactivateur ou les deux étant situés à l'intérieur de l'amorce, le désactivateur étant situé à l'extrémité 5' ou à proximité de celle-ci ; et ladite amorce doublement marquée subissant une modification détectable de la fluorescence lors de l'extension desdites une ou plusieurs amorces marquées pour donner ladite molécule d'acide nucléique, l'extension étant d'au moins 3 nucléotides.

3. Procédé selon la revendication 1 ou 2, dans lequel les étapes (a), (b) et (c) sont effectuées simultanément ou séparément dans un ordre quelconque.

4. Procédé selon la revendication 1 ou 2, dans lequel l'étape (c) est effectuée en présence d'oligonucléotides non incorporés marqués par fluorescence.

5. Procédé selon la revendication 1 ou 2, dans lequel aucunes étapes de traitement supplémentaires ne sont nécessaires entre les étapes (b) et (c) ou en même temps que l'étape (c), éventuellement les étapes de traitement supplémentaires étant choisis dans le groupe comportant l'électrophorèse sur gel, l'immobilisation du produit d'amplification et l'élimination par lavage de l'oligonucléotide non incorporé, la digestion ou le clivage de l'oligonucléotide, le traitement par 3'→5' exonucléase, la dénaturation et le traitement thermique.

6. Procédé selon la revendication 1 ou 2, dans lequel le désactivateur et le fluorophore sont séparés d'une distance telle que, lorsque l'oligonucléotide n'est pas lié à l'acide nucléique cible, le fluorophore est désactivé par le désactivateur et, lorsque l'oligonucléotide est lié à l'acide nucléique cible et étendu d'au moins trois nucléotides, le fluorophore n'est pas désactivé par le désactivateur.

7. Procédé selon la revendication 1, dans lequel la distance est comprise entre 3 et 20 nucléotides environ ou procédé de la revendication 2, dans lequel la distance est comprise entre 4 et 20 nucléotides environ.

8. Procédé selon la revendication 1, dans lequel la distance est comprise entre 6 et 19 nucléotides environ.

9. Procédé selon la revendication 1 ou 2, dans lequel le fluorophore est choisi dans le groupe comportant la fluorescéine, la 5-carboxy-fluorescéine (FAM), la 2'7'-diméthoxy-4'5'-dichloro-6-carboxyfluorescéine (JOE), la rhodamine, la 6-carboxyrhodamine (R6G), la N,N,N',N'-tétraméthyl-6-carboxyrhodamine (TAMRA), la 6-carboxy-X-rhodamine (ROX), l'acide 4-(4'-diméthylaminophénylazo)benzoïque (DABCYL) et l'acide 5-(2'-aminoéthyl)aminonaphtalène-1-sulfonique (EDANS) et/ou dans lequel le désactivateur est choisi dans le groupe comportant un désactivateur Black Hole®Quencher, un désactivateur Iowa Black®, un désactivateur Eclipse®Dark et un désactivateur DABCYL et un dérivé de ceux-ci.

10. Procédé selon la revendication 1, dans lequel le fluorophore est situé à l'intérieur dudit oligonucléotide et le désactivateur est situé à l'extrémité 5' dudit oligonucléotide, ou procédé selon la revendication 2, dans lequel le fluorophore est situé sur un nucléotide interne et le désactivateur est situé à l'extrémité 5' de l'amorce doublement marquée.

11. Procédé selon la revendication 1 ou 2, dans lequel ledit acide nucléique cible a une longueur de 15 à 100 nucléotides environ.

12. Procédé selon la revendication 1 ou 2, dans lequel la détection est réalisée à l'aide d'un instrument PCR en temps réel, de préférence un instrument spectrophotométrique PCR en temps réel.

13. Procédé selon la revendication 1 ou 2, dans lequel l'acide nucléique cible est choisi dans le groupe comportant l'ADN génomique, l'ARN, l'ADNc, l'ARNm et l'ADN synthétisé par voie chimique, éventuellement l'acide nucléique cible étant une séquence d'un agent pathogène infectieux, séquence génomique humaine de type sauvage, ou une mutation impliquée dans une maladie ou un trouble chez l'homme.

14. Procédé selon la revendication 1, comprenant en outre les opérations consistant à dénaturer le produit de l'étape (b) et laisser incuber dans des conditions suffisantes pour synthétiser une ou plusieurs molécules d'acide nucléique complémentaires de la totalité ou d'une partie desdites une ou plusieurs molécules d'acide nucléique cibles, lesdites une ou molécules d'acide nucléique de synthèse comprenant lesdits un ou plusieurs oligonucléotides, et répéter éventuellement une ou plusieurs fois la dénaturation et l'incubation.

15. Procédé de quantification ou détection d'une ou de plusieurs molécules d'acide nucléique cibles dans un échantillon lors de la synthèse d'acide nucléique, le procédé comprenant les opérations consistant à :
a) mélanger une ou plusieurs molécules d'acide nucléique cibles à une ou plusieurs amorces oligonucléotidiques simple brin complémentaires d'une partie desdites une ou plusieurs molécules d'acide nucléique cibles, chaque amorce étant un oligonucléotide marqué par fluorescence, marqué avec un fluorophore et un désactivateur ; un des fluorophore et désactivateur ou les deux étant situés dans une partie de l'amorce qui est complémentaire de la cible ; un des fluorophore et désactivateur ou les deux étant situés à l'intérieur de l'amorce, le désactivateur étant situé à l'extrémité 5' ou à proximité de celle-ci ;
b) incuber ledit mélange avec une polymérase dans des conditions suffisantes pour synthétiser une ou plusieurs molécules d'acide nucléique complémentaires de la totalité ou d'une partie desdites une ou plusieurs molécules d'acide nucléique cibles, lesdites une ou plusieurs molécules d'acide nucléique synthétisées comprenant lesdits un ou plusieurs oligonucléotides, l'extension desdites une ou plusieurs amorces d'au moins 3 nucléotides par ladite polymérase déclenchant de la fluorescence ; et
c) détecter la présence ou l'absence, ou quantifier la quantité, desdites une ou plusieurs molécules d'acide nucléique synthétisées par mesure de ladite fluorescence.

16. Oligonucléotide marqué par fluorescence comprenant à la fois un fluorophore et un désactivateur, le fluorophore étant fixé à l'intérieur du oligonucléotide, à l'extrémité 5' ou à proximité de l'extrémité 5', et le désactivateur étant fixé à l'intérieur du oligonucléotide, à l'extrémité 5' ou à proximité de l'extrémité 5', et le fluorophore et le désactivateur étant séparés d'une distance telle que, lorsque l'oligonucléotide est lié à un acide nucléique cible et étendu d'au moins trois nucléotides, le fluorophore n'est pas désactivé par le désactivateur et, lorsque l'oligonucléotide n'est pas lié à un acide nucléique cible, le fluorophore est désactivé par le désactivateur, la distance étant comprise entre 3 et 20 nucléotides ou entre 6 et 19 nucléotides.

17. Oligonucléotide selon la revendication 16, dans lequel le fluorophore est choisi dans le groupe comportant la fluorescéine, la 5-carboxyfluorescéine (FAM), la 2,7'-diméthoxy-dichloro-6-4'5'- carboxyfluorcscéine (JOE), la rhodamine, la 6-carboxyrhodamine (R6G), la N,N,N',N'-tétraméthyl-6-carboxyrhodamine (TAMRA), la 6-carboxy-X-rhodamine (ROX), l'acide 4-(4'-diméthylaminophénylazo)benzoïque (DABCYL), et l'acide 5-(2'-aminoéthyl)aminonaphtalène-1-sulfonique (EDANS) et/ou dans lequel le désactivateur est choisi dans le groupe comportant un désactivateur Black Hole Quencher®, un désactivateur Iowa Black®, un désactivateur Eclipse®Dark, un désactivateur DABCYL et un dérivés de ceux-ci.

18. Kit de détection ou de quantification d'une ou de plusieurs molécules d'acide nucléique cibles dans un échantillon lors de la synthèse d'acide nucléique, comprenant :
(a) une polymérase, et
(b) un oligonucléotide doublement marqué comprenant un fluorophore et un désactivateur, un des fluorophore et désactivateur ou les deux étant situés à l'intérieur de l'amorce, le désactivateur étant situé à l'extrémité 5' ou à proximité de celle-ci ; le désactivateur et le fluorophore étant séparés d'une distance telle que, lorsque l'oligonucléotide doublement marqué lié à la molécule d'acide nucléique n'est pas étendu, le fluorophore est désactivé par le désactivateur et, lorsque l'oligonucléotide doublement marqué lié à la molécule d'acide nucléique est étendu, le fluorophore n'est pas désactivé par le désactivateur et la distance étant comprise entre 3 et 20 nucléotides.

19. Composition comprenant :
(a) une polymérase, et
(b) un oligonucléotide doublement marqué comprenant un fluorophore et un désactivateur, un des fluorophore et désactivateur ou les deux étant situés à l'intérieur de l'amorce, le désactivateur étant situé à l'extrémité 5' ou à proximité de celle-ci ; le désactivateur et le fluorophore étant séparés d'une distance telle que, lorsque l'oligonucléotide doublement marqué lié à la molécule d'acide nucléique n'est pas étendu, le fluorophore est désactivé par le désactivateur et, lorsque l'oligonucléotide doublement marqué lié à la molécule d'acide nucléique est étendu, le fluorophore n'est pas désactivé par le désactivateur et la distance étant comprise entre 3 et 20 nucléotides.


### SEQUENCE LISTING

<110> GE, Wayne NG, Yue Ling
<120> QUANTITATIVE REAL-TIME PCR ASSAY USING FRET DUAL-LABELED PRIMERS
<130> LT00288 PCT
<140> Not Yet Assigned
   <141> 2011-08-19
<150> US 61/375318
   <151> 2010-08-20
<160> 35
<170> PatentIn version 3.5
<210> 1
   <211> 54
   <212> DNA
   <213> Equine herpesvirus 1
<400> 1
   atctggccgg gcttcaacca tccgtcaact actcgacgtt cgaggtgggt gacc 54
<210> 2
   <211> 20
   <212> DNA
   <213> Artificial Sequence
<220>
   <223> primer sequence
<400> 2
   actcgacgtt cgaggtgggt 20
<210> 3
   <211> 19
   <212> DNA
   <213> Artificial Sequence
<220>
   <223> primer sequence
<400> 3
   tgcaagctcc acccactgg 19
<210> 4
   <211> 19
   <212> DNA
   <213> Artificial Sequence
<220>
   <223> FRET primer sequence
<220>
   <221> misc_feature
   <222> (1)..(1)
   <223> Quencher molecule attached
<220>
   <221> misc_feature
   <222> (19)..(19)
   <223> FAM fluorophore attached
<400> 4
   ggtcacccac ctcgaacgt 19
<210> 5
   <211> 19
   <212> DNA
   <213> Artificial Sequence
<220>
   <223> FRET primer sequence
<220>
   <221> misc_feature
   <222> (1)..(1)
   <223> Quencher molecule attached
<220>
   <221> misc_feature
   <222> (12)..(12)
   <223> FAM fluorophore attached
<400> 5
   ggtcacccac ctcgaacgt 19
<210> 6
   <211> 19
   <212> DNA
   <213> Artificial Sequence
<220>
   <223> FRET primer sequence
<220>
   <221> misc_feature
   <222> (1)..(1)
   <223> BHQ1 quencher molecule attached
<220>
   <221> misc_feature
   <222> (3)..(3)
   <223> FAM fluorophore attached
<400> 6
   ggtcacccac ctcgaacgt 19
<210> 7
   <211> 18
   <212> DNA
   <213> Artificial Sequence
<220>
   <223> primer sequence
<400> 7
   atctggccgg gcttcaac 18
<210> 8
   <211> 27
   <212> DNA
   <213> Artificial Sequence
<220>
   <223> FRET primer sequence
<220>
   <221> misc_feature
   <222> (1)..(1)
   <223> Iowa Black FQ quencher attached
<220>
   <221> misc_feature
   <222> (2)..(27)
   <223> Internal fluorophore molecule attached
<400> 8
   tgatgcagtg caagctccac ccactgg 27
<210> 9
   <211> 15
   <212> DNA
   <213> Artificial Sequence
<220>
   <223> probe sequence
<400> 9
   atccgtcaac tactc 15
<210> 10
   <211> 20
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (12)..(12)
   <223> FAM fluorophore attached
<400> 10
   tggtcccgtc ctcatctcag 20
<210> 11
   <211> 20
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (17)..(17)
   <223> FAM fluorophore attached
<400> 11
   tggtcccgtc ctcatctcag 20
<210> 12
   <211> 20
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (4)..(4)
   <223> FAM fluorophore attached
<400> 12
   tggtcccgtc ctcatctcag 20
<210> 13
   <211> 19
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (14)..(14)
   <223> FAM fluorophore attached
<400> 13
   tggtcccgtc tcatctcag 19
<210> 14
   <211> 20
   <212> DNA
   <213> Artificial Sequence
<220>
   <223> primer sequence
<400> 14
   ccagttcaga atcggacgtt 20
<210> 15
   <211> 19
   <212> DNA
   <213> Artificial Sequence
<220>
   <223> primer sequence
<400> 15
   gctgccttgc cggaaatta 19
<210> 16
   <211> 20
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (10)..(10)
   <223> FAM fluorophore attached
<400> 16
   aaagacgcct accctgttga 20
<210> 17
   <211> 20
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (18)..(18)
   <223> FAM fluorophore attached
<400> 17
   aaagacgcct accctgttga 20
<210> 18
   <211> 20
   <212> DNA
   <213> Artificial Sequence
<220>
   <223> primer sequence
<220>
   <221> misc_feature
   <222> (1)..(1)
   <223> BHQ1 quencher attached
<220>
   <221> misc_feature
   <222> (15)..(15)
   <223> FAM fluorophore attached
<400> 18
   aaagacgcct accctgttga 20
<210> 19
   <211> 20
   <212> DNA
   <213> Artificial Sequence
<220>
   <223> FRET primer sequence
<220>
   <221> misc_feature
   <222> (1)..(1)
   <223> BHQ1 quencher attached
<220>
   <221> misc_feature
   <222> (17)..(17)
   <223> FAM fluorophore attached
<400> 19
   aaagacgcct accctgttga 20
<210> 20
   <211> 20
   <212> DNA
   <213> Artificial Sequence
<220>
   <223> primer sequence
<400> 20
   tggtcccgtc ctcatctcag 20
<210> 21
   <211> 20
   <212> DNA
   <213> Artificial Sequence
<220>
   <223> primer sequence
<400> 21
   ccagttcaga atcggacgtt 20
<210> 22
   <211> 19
   <212> DNA
   <213> Artificial Sequence
<220>
   <223> primer sequence
<400> 22
   gctgccttgc cggaaatta 19
<210> 23
   <211> 20
   <212> DNA
   <213> Artificial Sequence
<220>
   <223> primer sequence
<400> 23
   aaagacgcct accctgttga 20
<210> 24
   <211> 28
   <212> DNA
   <213> Artificial Sequence
<220>
   <223> probe sequence
<220>
   <221> misc_feature
   <222> (1)..(1)
   <223> FAM fluorophore attached
<220>
   <221> misc_feature
   <222> (28)..(28)
   <223> NFQ quencher attached
<400> 24
   agccatgaag aatgccgtct atcacaca 28
<210> 25
   <211> 28
   <212> DNA
   <213> Artificial Sequence
<220>
   <223> probe sequence
<220>
   <221> misc_feature
   <222> (1)..(1)
   <223> FAM fluorophore attached
<220>
   <221> misc_feature
   <222> (28)..(28)
   <223> NFQ quencher attached
<400> 25
   tccatagttc ctggtttgta atgtgccg 28
<210> 26
   <211> 31
   <212> DNA
   <213> Artificial Sequence
<220>
   <223> primer sequence
<220>
   <221> misc_feature
   <222> (1)..(1)
   <223> FAM fluorophore attached
<220>
   <221> misc_feature
   <222> (31)..(31)
   <223> BHQ1 quencher attached
<400> 26
   cgctcgttac tcgtcaggca ctcggtgagc g 31
<210> 27
   <211> 15
   <212> DNA
   <213> Artificial Sequence
<220>
   <223> primer sequence
<400> 27
   ccaccctggc gctcg 15
<210> 28
   <211> 18
   <212> DNA
   <213> Artificial Sequence
<220>
   <223> primer sequence
<400> 28
   gccgggcttc aaccatcc 18
<210> 29
   <211> 27
   <212> DNA
   <213> Artificial Sequence
<220>
   <223> primer sequence
<400> 29
   gcttcaacca tccgtcaact actcgac 27
<210> 30
   <211> 26
   <212> DNA
   <213> Artificial Sequence
<220>
   <223> primer sequence
<400> 30
   aaccatccgt caactactcg acgttc 26
<210> 31
   <211> 24
   <212> DNA
   <213> Artificial Sequence
<220>
   <223> primer sequence
<400> 31
   tccgtcaact actcgacgtt cgag 24
<210> 32
   <211> 22
   <212> DNA
   <213> Artificial Sequence
<220>
   <223> primer sequence
<400> 32
   caactactcg acgttcgagg tg 22
<210> 33
   <211> 39
   <212> DNA
   <213> Artificial Sequence
<220>
   <223> primer sequence
<400> 33
   gccagtgaat tattaatacg actcactata gggagaaga 39
<210> 34
   <211> 20
   <212> DNA
   <213> Artificial Sequence
<220>
   <223> primer sequence
<400> 34
   tcgcgcgttt cggtgatgac 20
<210> 35
   <211> 16
   <212> DNA
   <213> Artificial Sequence
<220>
   <223> probe sequence
<220>
   <221> misc_feature
   <222> (1)..(1)
   <223>. FAM fluorophore attached
<220>
   <221> misc_feature
   <222> (16)..(16)
   <223> MGB fluorophore attached
<400> 35
   atccgtcgac tactcg 16